# EUROPEAN PATENT SPECIFICATION

(11) **EP 3 105 211 B1**
(45) Date of publication and mention of the grant of the patent: **20.12.2017**
(21) Application number: 15703787.0
(22) Date of filing: 12.02.2015
(51) Int. Cl.: C07D 253/06, C07D 513/08, C08G 83/00

(54) **POLYMERS**
POLYMERE
POLYMÈRES

(30) Priority: 14.02.2014 GB 201402646
(43) Date of publication of application: 21.12.2016
(73) Proprietor: ETH Zurich, 8092 Zürich (CH)
(72) Inventor: KORY, Max Jonathan, CH-8093 Zurich (CH); SCHLUETER, Dieter A., CH-8093 Zurich (CH)
(74) Representative: Schaad, Balass, Menzl & Partner AG
(86) International application number: PCT/EP2015/052934
(87) International publication number: WO 2015/121336

(56) References cited:
- KISSEL P. ET AL.: "A two-dimensional polymer prepared by organic synthesis", NATURE CHEMISTRY, vol. 4, 5 February 2012 (2012-02-05), pages 287-291, XP002738217, DOI: 10.1038/nchem.1265 cited in the application
- MAX J. KORY ET AL: "Facile Synthesis and Theoretical Conformation Analysis of a Triazine-Based Double-Decker Rotor Molecule with Three Anthracene Blades", CHEMISTRY - A EUROPEAN JOURNAL, vol. 20, no. 23, 2 June 2014 (2014-06-02), pages 6934-6938, XP055181747, ISSN: 0947-6539, DOI: 10.1002/chem.201400364

## Description

The present invention relates to polymers comprising as monomer units one or more compounds having at least one triazine or at least one pyrimidine core.

Synthetic polymers are widely used materials, as attested by a production of more than 200 millions of tons per year, and are typically composed of linear repeat units. The characteristics of the polymers depend, among others, on the monomer unit and the molecular weight of the polymer.

A two-dimensional polymer is a sheet-like macromolecule having laterally connected repeat units with end groups along all edges. It is a one monomer unit thick, covalent network with a translational periodicity. Due to its shape and periodical network structure, a two-dimensional polymer exhibits unique structural and physical properties. A two-dimensional polymeric material may comprise several layers of two-dimensional polymers.

The natural representative of a two-dimensional polymer is graphene. While graphene can meanwhile routinely be prepared by exfoliation of graphite, graphite oxide, or by chemical vapor deposition rational organic synthesis of artificial two-dimensional polymers still remains a major challenge in chemistry. The main reason for this lies in the difficulty to control the extremely complex bond formation process during polymerization.

In Nature Chemistry, Vol. 4, April 2012, 287-291, a two-dimensional polymer prepared by organic synthesis is disclosed. The disclosed monomer has three photoreactive 1,8-diethynylanthrylene units and three terphenylene bridges.

US 2004/0227605 discloses a molecular system having at least three branches, with one end of each branch connected to an immobile junction unit, with two of the branches on one side of the junction unit and with at least one other branch on the opposite side of the junction unit. However, said molecular system is not described as a monomer unit for a polymer.

C. J. Lee et al Tetrahedron Letters, 2002, 43, 3863-3866, disclose liquid crystal materials whose molecular structures consist of a disc-like 1,3,5-triazine unit as a central core and three rod-like azobenzenes as the peripheral arms. No two-dimensional polymerization is possible with such a monomer structure.

P. Kissel et al. European Journal 2009, 15, 8955-8960 disclose a synthesis of a macrocycle with three 1,8-anthrylene units. Said macrocylces could be good monomers for two dimensional polymers. However, the synthesis of said monomer units is very expensive and time consuming.

Bhola et al. disclose a two-dimensional polymer from the anthracene dimer and triptycene motifs in J. Am. Chem. Soc. 2013, 135, 14134-14141. The polymer is obtained by a photoinduced polymerization, whereby the monomers are in the crystalline state.

However, the synthesis of said monomer units is very difficult, involves a significant number of synthesis steps and a scale up would be too expensive.

The problem of the present invention is to provide polymers made by monomer units which allow for one or two-dimensional synthesis on a more economic scale.

The problem is solved by the polymer of claim 1 and the method for preparing said polymer according to claim 7. Further preferred embodiments are subject of dependent claims.

Surprisingly, it was found that a polymer according to the present invention can be obtained by an efficient route. It comprises as monomer unit one or more of the below mentioned compounds comprising at least one triazine or pyrimidine core. Said core allows a fast and cheap synthesis due to its easy functionalization by employing cyanuric chloride, cyanuric fluoride, 2,4,6-trichloro-pyrimidine or 2,4,6-trifluoro-pyrimidine. The monomer units comprise photoreactive groups, which can be converted into sheet-like polymers under UV-radiation or by thermally induced polymerization.

Within the context of the present invention the expression "polymer" encompasses molecular structures, which extend in one or two dimensions. For a molecular structure in two dimensions "polymer" encompasses one single monolayer as well as a polymeric material comprising more than one monolayer. In addition, the polymer comprises at least 2 monomer units, but may also comprise several thousand or more monomer units. The polymer may be in all different states, such as in a crystalline, a semicrystalline or an amorphous state. In addition, the polymer within the context of the present invention may be fully or partially polymerized. The polymer can be a homopolymer, that is a polymer consisting of one type of monomer unit or can be a co-polymer, that is a polymer comprising at least two different types of monomer units.

The monomer unit of the polymer according to the present invention is a compound selected from the group consisting of 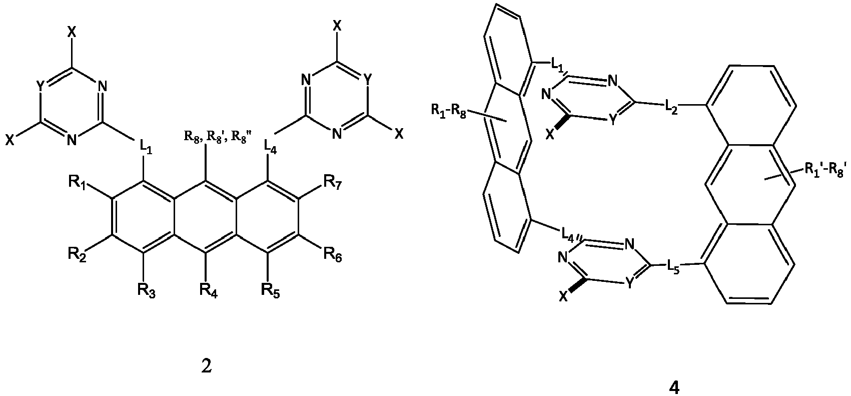 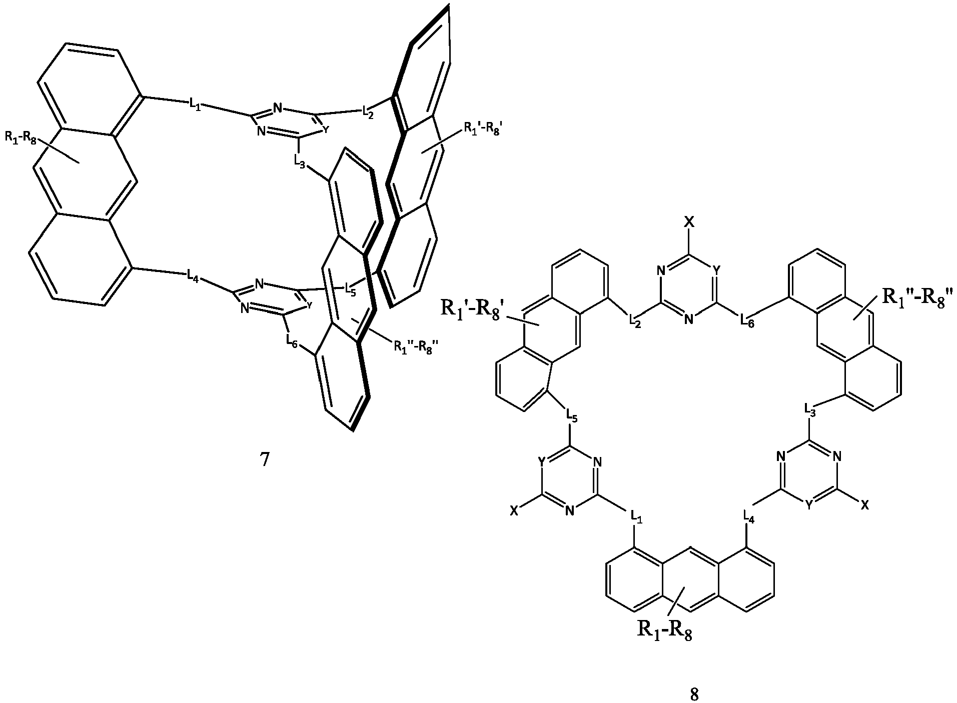 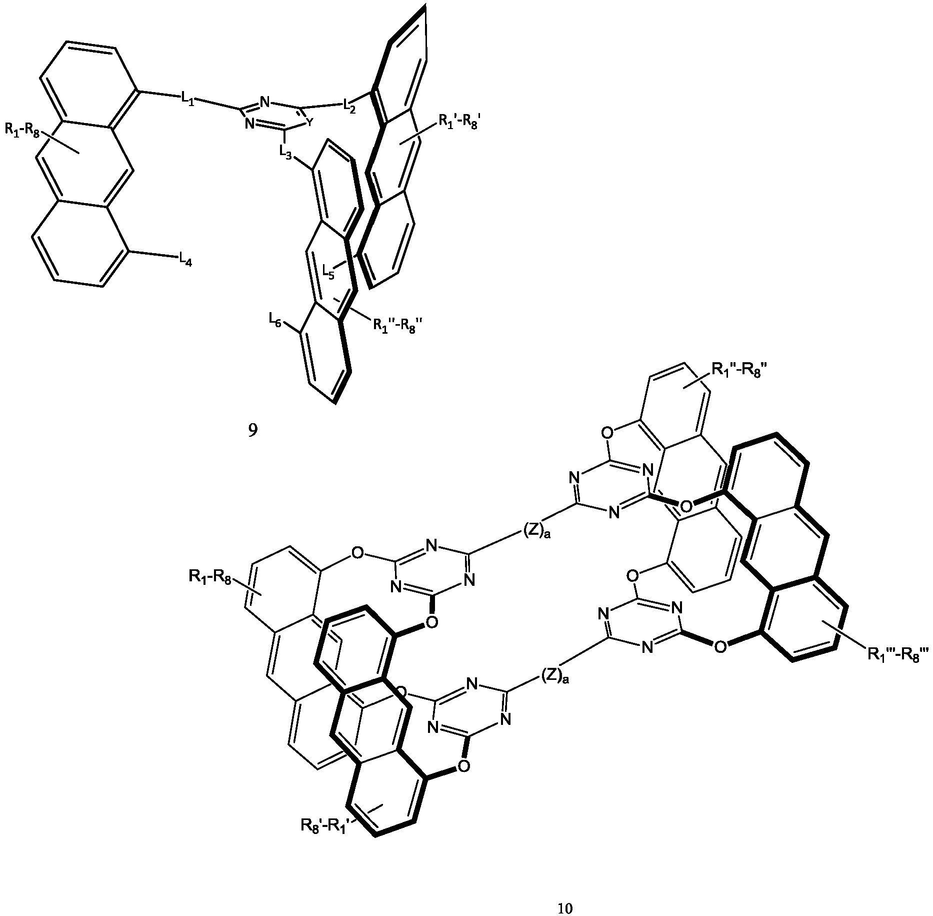 wherein the residues R₁ to R₈ of the first anthracene moiety, R₁' to R₈' of the second anthracene moiety, R₁" to R₈" of the third anthracene moiety and R₁'" to R₈'" of the forth anthracene moiety in formulas 4 (two anthracene moieties), 7 (three anthracene moieties), 8 (three anthracene moieties), 9 (three anthracene moieties) and 10 (four anthracene moieties) are arranged as follows 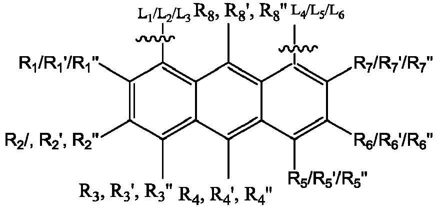 and in formulas 2, 4, 7, 8, 9 and 10
R₁, R₁', R₁", R₁"', R₂, R₂', R₂", R₂"', R₃, R₃', R₃"', R₃"', R₄, R₄', R₄", R₄"', R₅, R₅', R₅", R₅'", R₆, R₆', R₆", R₆"', R₇, R₇', R₇", R₇"', R₈, R₈', R₈" and R₈" are independently from each other hydrogen, deuterium or a linear or branched alkyl group having 1 to 8 carbon atoms, preferably selected from the group of methyl, ethyl or propyl,
each of R₁ and R₂, R₁' and R₂', R₁" and R₂", R₁'" and R₂"', R₂ and R₃, R₂' and R₃', R₂" and R₃", R₂'" and R₃'", R₅ and R₆, R₅' and R₆', R₅" and R₆", R₅'" and R₆"', R₆ and R₇, R₆' and R₇', R₆" and R₇", R₆'" and R₇"' may form independently from each other a six-membered aromatic ring which may optionally be substituted with a linear or branched alkyl group having 1 to 16 carbon atoms, preferably selected from the group of methyl, ethyl or propyl residues, and
L₁, L₂, L₃, L₄, L₅ and L₆ are independently from each other oxygen, sulphur, acetylene, or NR₉, and R₉ is hydrogen or methyl, and
X is either chlorine or fluorine,
Y is nitrogen or CH, preferably nitrogen, and
Z is NH, O, S, an alkyne group having 1 to 16 carbon atoms, alkylene group having 1 to 16 carbon atoms, arylene group having 2 to 16 carbon atoms, heteroarylene group having 2 to 16 carbon atoms or oligo (ethylene glycol) group having 1 to 10 CH₂CH₂O units, and (a) may be 0 or 1. If (a) is 0 the two triazine cores in 10 are directly connected.

If, for example, the polymer according to the present invention comprises as monomer unit the compound of formula 2 or 4 the polymerization can occur in one dimension, since said monomer units can be arranged in an uniaxial state. In contrast thereto, if the polymer according to the present invention comprises for example the compounds of formula 7, 8, 9 or 10, the polymerization can occur in two dimensions, since said monomer units can be arranged in a biaxial state.

Preferably, the polymer comprises as monomer unit a compound of formula 7, wherein all residues R₁, R₁', R₁", R₁"', R₂, R₂', R₂", R₂"', R₃, R₃', R₃", R₃"', R₄, R₄', R₄", R₄"', R₅, R₅', R₅", R₅"', R₆', R₆', R₆", R₆"', R₇, R₇', R₇", R₇"', R₈', R₈', R₈", R₈"', L₁, L₂, L₃, L₄, L₅, L₆, and Y have the same definition as mentioned above.

The polymer according to the present invention preferably comprises as monomer unit a compound of formula 2, 4, 7, 8, 9 and 10, and most preferably a compound of formula 7, wherein R₄, R₄', R₄" and R₄"' as well as R₈, R₈', R₈", R₈"' are all hydrogen. Due to the presence of the hydrogen atoms in position 4 and 8, it is easier to bring the compounds in an uniaxial or biaxial oriented state, which is favourable for the later solid state polymerization. Most preferably R₁, R₁', R₁", R₁"', R₂, R₂', R₂", R₂"', R₃, R₃', R₃", R₃"', R₄, R₄', R₄", R₄"', R₅, R₅', R₅", R₅"', R₆, R₆', R₆", R₆"', R₇, R₇', R₇", R₇"', R₈, R₈', R₈" and R₈" are all hydrogen. By the presence of hydrogens in all positions (1, 2, 3, 4, 5, 6, 7 and 8) of the anthracene blades, the educts are easy accessible.

The polymer according to the present invention preferably comprises as monomer unit a compound as mentioned above, and, most preferably, a compound of formula 7, wherein L₁, L₂, L₃, L₄, L₅ and L₆ are all oxygen or all sulphur, preferably, are all oxygen. Most preferably, R₄, R₄', R₄" and R₄"' as well as R₈, R₈', R₈", R₈"' are all hydrogen and L₁, L₂, L₃, L₄, L₅ and L₆ are all oxygen. Most preferably R₁, R₁', R₁", R₁"', R₂, R₂', R₂", R₂"', R₃, R₃', R₃", R₃"', R₄, R₄', R₄", R₄"', R₅, R₅', R₅", R₅"', R₆, R₆', R₆", R₆"', R₇, R₇', R₇", R₇"', R₈, R₈', R₈" and R₈'" are all hydrogen as well.

The monomer units of the polymer of the present invention preferably comprise only triazine cores, that is, Y is nitrogen. Most preferably in the monomer units comprising only triazine cores and no pyrimidine cores, the residues R₁, R₁', R₁", R₁"', R₂, R₂', R₂", R₂"', R₃, R₃', R₃", R₃"', R₄, R₄', R₄", R₄"', R₅, R₅', R₅", R₅'", R₆, R₆', R₆", R₆"', R₇, R₇', R₇", R₇"', R₈, R₈', R₈" and R₈"' are all hydrogen and L₁, L₂, L₃, L₄, L₅ and L₆ are all oxygen or all sulphur, preferably all oxygen. Due to the presence of the triazine cores it is particularly easy to generate the desired supramolecular architecture required for solid-state polymerization reaction through ability of the triazine cores to undergo supramolecular interactions.

Most preferably, at least one monomer unit of the polymer according to the present invention is the compound of the formula 7a 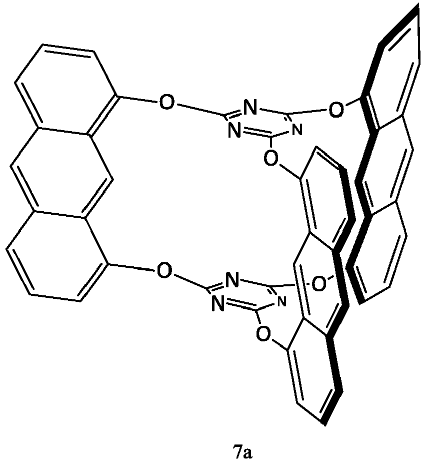 which corresponds to the compound of formula 7 wherein L₁, L₂, L₃, L₄, L₅ and L₆ are all oxygen, R₁, R₁', R₁", R₂, R₂', R₂", R₃, R₃', R₃", R₄, R₄', R₄", R₅, R₅', R₅", R₆, R₆', R₆", R₇, R₇', R₇", R₈, R₈', and R₈" are all hydrogen and Y is nitrogen.

In a preferred embodiment of the present invention the polymer is a homopolymer, that is, the monomer units of the polymer are identical. The characteristics of such a homopolymer are the same over the entire length of the polymer. A preferred homopolymer is a polymer having as monomer unit the compound of formula 7a.

In another embodiment of the present invention, the polymer is a copolymer comprising several different monomer units, preferably two or three different monomer units having a different chemical formula. The first monomer unit is selected from the group consisting of a compound of formula 2, 4, 7, 8, 9 and 10, most preferably compound of formula 7a. The different types of monomer units may be randomly distributed or may be distributed in a regular manner.

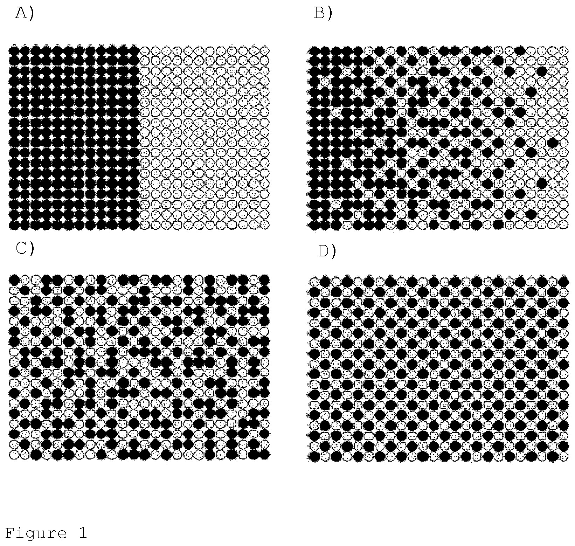

For example, the polymer can comprise a first monomer unit and a second monomer unit, wherein the first monomer unit is selected from the group consisting of a compound of formula 2, 4, 7, 8, 9 and 10, preferably compound 7a, and the second monomer unit is selected from a group consisting of a compound of formula 2, 4, 7, 8, 9 and 10, but is different from the first monomer unit. For lateral polymerization, the first and the second monomer unit may be randomly distributed (for example as shown in Figures 1B) and 1C)) or may be distributed in a regular manner (for example as shown in Figures 1A) and 1D)). In Figures 1A to 1D black dots stand for the first monomer unit and white dots stand for the second monomer unit.

Alternatively, the monomer unit of the first monomer unit is the same as the second monomer unit but differs in the residue. That is at least one of the residues R₁, R₁', R₁", R₁"', R₂, R₂', R₂", R₂"', R₃, R₃', R₃", R₃"', R₄, R₄', R₄", R₄"', R₅, R₅', R₅", R₅'", R₆, R₆', R₆", R₆"', R₇, R₇', R₇", R₇"', R₈, R₈', R₈", R₈"', L₁, L₂, L₃, L₄, L₅, L₆, X, Y or Z is different from the one in the second polymer unit. For example the first monomer unit of the polymer according to the present invention can be a compound of formula 7, wherein R₁, R₁', R₁", R₂, R₂', R₂", R₃, R₃', R₃", R₄, R₄', R₄", R₅, R₅', R₅", R₆, R₆', R₆", R₇, R₇', R₇", R₈, R₈', R₈" are all hydrogen, L₁, L₂, L₃, L₄, L₅, L₆, X are all oxygen and Y is nitrogen, whereas the second monomer unit of the polymer is a compound of formula 7, wherein R_{1,} R₁', R₁", R₂, R₂', R₂", R₃, R₃', R₃", R₄, R₄', R₄", R_{5,} R₅', R₅", R₆, R₆', R₆", R₇, R₇', R₇", R₈, R₈', R₈" are all hydrogen, L₁, L₂, L₃, L₄, L₅, X are all oxygen and Y is nitrogen and L₆, as only difference, is sulphur. However, it is also possible, that the first and the second monomer unit differ in more than one residue, for example they can have the same chemical formula but differ in the residues L₁, L₂, L₃, L₄, L₅, L₆, said residues being in one monomer unit all oxygen and in the other monomer unit all sulphur. Also in this embodiment for lateral polymerization, the first and the second monomer unit may be randomly distributed (for example as shown in Figures 1B) and 1C)) or may be distributed in a regular manner (for example as shown in Figures 1A) and 1D)). In Figures 1A to 1D black dots stand for the first monomer unit and white dots stand for the second monomer unit.

In another alternative embodiment of the present invention, the polymer can comprise a first monomer unit which is selected from the group consisting of compound of formula 2, 4, 7, 8, 9 and 10, and as a second monomer unit a monofunctional or bifunctional nucleophilic compound. The core (K) of such a monofunctional or bifunctial nucleophilic compound may be a linear or branched alkyl chain having 1 to 10 carbon atoms, a linear or branched alkylene chain having 1 to 10 carbon atoms, a linear or branched arylene chain having 2 to 12 carbon atoms, an oligo (ethylene glycol) chain (OEG) having 1 to 10 carbon atoms. The one or two functional groups are, preferably, terminal groups; however, it is also possible, that they are at another position of the core chain. The functional groups are preferably primary amines (such as compound formula 100) or secondary amines (such as compound of formula 101, wherein L is preferably methyl, ethyl or propyl), alcohols (such as compound of formula 102), acrylate (such as compound of formula 103), methylacrylate (such as compound of formula 104) or maleinimide (such as compound of formula 105).

HO-(K)ₙ-OH 100

H₂N-(K)ₙ-NH₂ 101

HLN-(K)ₙ-NLH 102

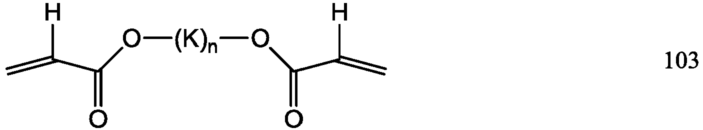

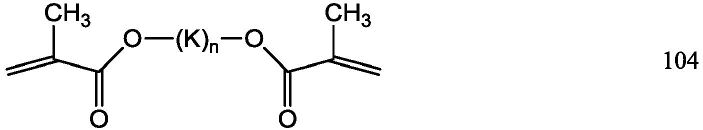

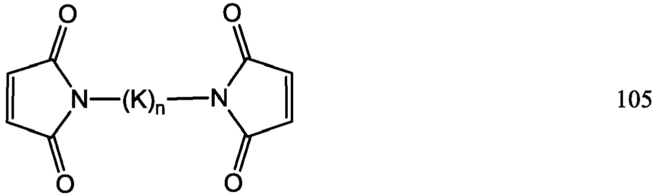

The two functional groups may be the same or different from each other (not shown in the above scheme). Most preferably the bifunctional nucleophilic compound is selected from the group consisting of methyldiamine, ethyldiamine, n-propyldiamine, n-butyldiamine, n-pentyldiamine, methandiol, ethandiol, propandiol, butandiol and pentandiol, poly(ethylene)glycol diacrylate, di(ethylene)glycol diacrylate, N,N'-(o-phenylene)dimaleimide, N,N'-(m-phenylene)dimaleimide. Also in this embodiment for lateral polymerization, the first and the second monomer unit may be randomly distributed (for example as shown in Figures 1B) and 1C)) or may be distributed in a regular manner (for example as shown in Figures 1A) and 1D)). In Figures 1A to 1D black dots stand for the first monomer unit and white dots stand for the second monomer unit.

Alternatively, the polymer can comprise a first monomer unit which is selected from a the group consisting of compound of formula 2, 4, 7, 8, 9 and 10, preferably compound 7a, and as a second monomer unit a dienophile, that is a compound which is capable of carrying out a [2+2] or [2+4] cycloaddition, preferably a fullerene, most preferably a C60-fullerene or a C70-fullerene. Also in this embodiment for lateral polymerization, the first and the second monomer unit may be randomly distributed (for example as shown in Figures 1B) and 1C)) or may be distributed in a regular manner (for example as shown in Figures 1A) and 1D)). In Figures 1A to 1D black dots stand for the first monomer unit and white dots stand for the second monomer unit.

In order to modify the characteristics and/or the processability of the polymer and the polymer material according to the present invention as well as to lower the costs, the polymer may be blended with a thermoplastic polymer. Preferably, the thermoplastic polymer is selected from the group consisting of polyamides, polyesters, polypropylene, polyethylene, polyarylene sulphides, polyarylene oxides, polyimides, polyetherketones, polyetheretherketones, polyoxymethylenes and polyetherimides, polyurethanes, rubbers, polycyanurates, polyaramids, epoxy resins and any copolymers and mixtures thereof. All different concentration ranges of the blend components are possible. However, preferably, such a polymer blend comprises 20 to 60 % by weight of the polymer according to the present invention and 40 to 80 % by weight of the thermoplastic polymer. Alternatively, it is also possible that the polymer according to the present invention is enclosed in a polymer jacket or polymer shell, said polymer jacked being made of a thermoplastic polymer. Alternatively, the polymer according to the present invention may be used as additive in a thermoplastic polymer as mentioned above at a concentration from 0.1 to 99.9% by weight of the thermoplastic polymer.

Preferably, the polymer according to the present invention is prepared by irradiating the monomer units in an uniaxial or biaxial oriented state with ultraviolet radiation to form the polymer, by thermally induced polymerization or by pressure induced polymerization. Within the context of the present invention a monomer unit in an uniaxial oriented state stands for a monomer unit having one axis along which anthracene moieties are arranged, whereas for a monomer unit in a biaxial oriented state anthracene moieties are arranged within a plane spanned by two orthogonal axes, enabling lateral polymerization. In order to polymerize the monomer units according to the present invention, the anthracene blades of the monomer unit stack preferably face-to-face to its neighbouring monomer units, which allows an efficient photopolymerization. The polymerization of the monomer units is facilitated by the pre-organization of the monomer units, which helps to reduce or prevent random cross-linking. In addition, due to the orientation of the monomer units, any undesired molecular motion can be supressed and therefore ensure lateral polymerization. The irradiation of the monomer units is, preferably, carried out at a wavelength of 320 to 600 nm, more preferably 420 to 500 nm and still more of 465 nm. The irradiations are preferably carried out under an inert atmosphere such as argon or nitrogen to supress oxidation of the anthracene units, alternatively the crystals can be covered with a UV transparent, oxygen impermeable packaging. Alternatively, the polymerization can be carried out as thermally induced polymerization by heating the oriented monomers to a temperature of up to 500°C, preferably to a temperature of 300°C to 480°C. In yet another way, polymerization can be carried out by pressure induced polymerization, that is by subjecting the oriented monomers to pressures of up to 10 GPa, preferably to pressures of 1 GPa to 5 GPa. Polymerization may be as well carried out by combining the three aforementioned methods.

Monomer units according to the present invention which are in an uniaxial or biaxial oriented state can be obtained by forming crystals or co-crystals from a solvent or a solvent mixture. Within the context of the present invention a crystal is a crystal comprising one type of monomer units, whereas a co-crystal comprises two or more types of monomer units. Both, that is crystals as well as co-crystals, may additionally comprise solvent molecules. Of course, crystallization is a preferred method, since it is cheap and can be carried out in large quantities. Suitable solvents have the capability to dissolve the monomer units according to the present invention to reach concentrations of 1 mg/ml to 150 mg/ml at or below their boiling points. Especially good results could be obtained with the solvent 2-cyanopyridine. The size and shape of the crystals may be controlled by gradient-cooling of the corresponding hot solution of the solvent comprising the monomer units.

Crystallization from solvents or solvent mixtures can also lead to crystals or co-crystals with domain formation, whereby the polar axes of the neighboring domains are inverted, that is, they are opposite to each other. Through crystallization under an electric field domain formation may be controlled.

Therefore, it is possible to engineer a scaffold of robust crystals, said scaffold having, for example, a size from 3 µm to 4 cm, which allows afterwards the production of the corresponding two-dimensional polymers.

Monomer unit 7, and in particular monomer unit 7a, shows a specific interaction with the solvent 2-cyanopyridine. It leads to the face-to-face stacking of the anthracene moieties of neighbouring 7a monomer units. The solvent molecule and the monomer unit 7a form a network-like arrangement comprising a structural unit. One third of the monomer units together with the solvent molecules form a template, that allows the remaining two third of the monomer units to assume a packing that, by the action of light is converted into a van-der-Waals crystal of two-dimensional polymer in the solid state. Said template fills a cavity with a volume in the range of 1300-to 1700 A³. By co-crystallization it is possible to fill this cavity also with other compounds and with this other also with other monomer units, for example with C60, C70 fullerenes.

The crystals of the monomer units can be isolated for instance by centrifugation before irradiation or irradiated without isolation.

Alternatively, the monomer units of the polymer according to the present invention can also be organized by forming a film, such as a Langmuir-Blodgett film of the monomer units. Such a Langmuir-Blodgett film contains at least one layer of the monomer units, deposited from the surface of a liquid, such as water, onto a solid substrate by immersing the solid substrate into or from the liquid. Suitable solid substrates are glass plates, metal plates, silicon wafers, mica or highly ordered pyrolytic graphite and the like, as well as flexible plastic films.

As another alternative, the polymer according to the present invention can be prepared by polymerization of a thin film of the monomer at a gas-liquid interface (for example air to water) or on a solid substrate. Thus, in order to facilitate the organization of the monomer units, a solution comprising monomer units as described above in one or more solvents, for instance 1,2-dichloroethane, is layered on the surface of water, or on a solid substrate such as glass plates, metal plates, silicon wafers, mica or highly ordered pyrolytic graphite and the like, as well as flexible plastic films. The solvents may be evaporated.

As a further alternative, the monomer units of the polymer of the present invention can be sublimed to form crystals, that is, as a first step the monomer units are heated at a temperature of 50° to 600°C, for example between 50°C to 350°C, under reduced pressure of 10⁻⁸ mbar to atmospheric pressure allowing the monomer units to sublime and, in a second step, the vapour thus obtained condenses on a cold surface forming crystals.

Alternatively, it is also possible to obtain an oriented layer of the monomer units according to the present invention by vapour deposition of the monomer units onto a substrate. Preferably, the vapour deposition is carried out at reduced pressure.

In addition, the monomer units can be sublimed, for example, by heating, and subsequently beaming them onto a solid substrate thus forming an oriented layer. Suitable solid substrates may be glass plates, metal plates, silicon wafers, mica, highly ordered pyrolytic graphite and the like as well as flexible plastic films.

The polymers of the present invention may be blended with a thermoplastic polymer as already mentioned above. Several types of blends are possible. The at least two components, that is at least one thermoplastic polymer selected from the group consisting of polyamides, polyesters, polypropylene, polyethylene, polyarylene sulfides, polyarylene oxides, polyimides, polyetherketones, polyetheretherketones, polyoxymethylenes and polyetherimides, polyurethanes, rubbers, polycyanurates, polyaramides, epoxy resins or copolymers and blends thereof and at least one polymer according to the present invention are mixed together.

In another embodiment of the present invention the polymer is post-modified by functionalization. During crystallisation the monomer units arrange themselves in an uniaxial or biaxial oriented state, the presence of additional functional groups may here be disturbing. However, in some cases it is advantageous to modify the polymer according to the present invention, for example, for a better polymer processing or for increasing the compatibility with other polymers, such as the thermoplastic polymers mentioned above. The post-modification can be carried out, for example, by alkylating one or more nitrogen atoms in the triazine cores or by hydrogenolysis. A post-modification of the polymers also allows a polymerization in three dimensions.

The polymer according to the present invention either alone or in form of a blend together, may be processed by spin coating, dip coating, drop casting, laminating, spray coating, melt extrusion or reaction injection molding. Of course other conventional techniques known to the person skilled in the art may also be used.

Although the polymer crystal according to the present invention is substantially insoluble in most common solvents, the immersion of the polymer crystal in some solvents, such as N-Methyl-2-pyrrolidon (NMP), gamma-butyrolactone, 2-cyanopyridine or acids such as trifluoro acetic acid or methyl sulfonic acid induces exfoliation into thin sheets. The polymeric material according to the present invention can be exfoliated into thin sheets with thickness 1 nm to 4 µm, preferably 1 nm to 1 µm. Alternatively, the sheets can be delaminated by micromechanical exfoliation using adhesive tape, such as scotch tape, whereby the polymer crystals are sprinkled onto the adhesive tape, which is subsequently repetitively folded onto itself and peeled apart, a protocol broadly utilized for the exfoliation of graphite to graphene, or the obtainment of e.g. thin MoS₂ sheets. If desired, the thin sheets comprising the polymer according to the present invention can be laminated to other polymer surfaces, such as to a polypropylene surface, to a polyethylene surface or to a poly(ethylene terephthalate) (PET) surface.

The polymer according to the present invention can be used as material in optoelectronic devices, light emitting diodes, laser diodes, organic transistors, capacitors, sensors, supercapacitors, dielectrics, high-k dielectrics, holographic devices, energy storage applications, batteries, photovoltaic devices, photonic crystal devices, magnetic devices, ferroelectric devices, piezoelectric devices, pyroelectric devices transparent conductive coatings, nucleating agents, clarifying agents, nonlinear optical devices, wear resistant coating, lubricating coating, protein repellent coating, catalyst support, scratch resistance coating, chiral surfaces, organic superconductor, photocatalysis, data storage applications, packaging, membranes, gas separation membranes or as desalination membrane. For said applications the polymer can be in the bulk, non-exfoliated state or it is exfoliated. In addition, the polymer according to the present invention may be used as polymer additive to enhance desired properties of the bulk polymer, that is, for example a thermoplastic polymer.

A further aspect of the present invention relates to a method for preparing the monomer unit of formula 7, and in particular to a method for preparing the monomer unit of formula 7a.

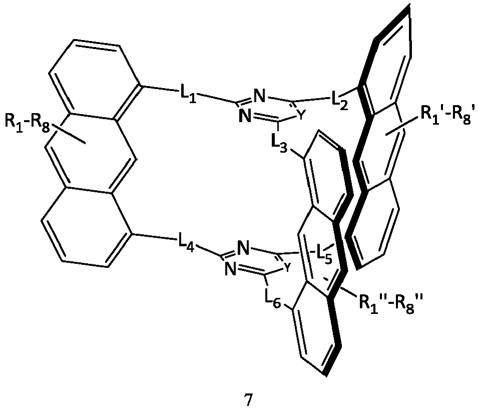

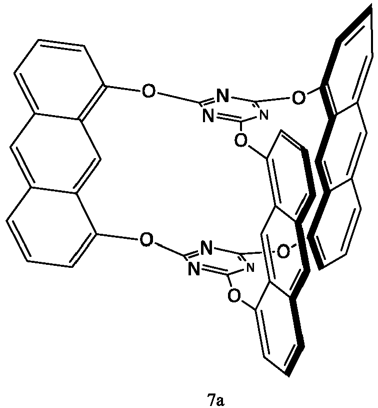

The reaction of the triazine (Y is N) or pyrimidine (Y is CH) based monomers is, preferably, carried out via stepwise substitution of the chlorine atom of cyanuric chloride or the fluorine atoms of cyanuric fluoride, or the chlorine atom of 2,4,6-trichloro-pyrimidine, or the fluorine atom of 2,4,6-trifluoro-pyrimidine, which reliably provides the oxygen-linked triazine monomer and the pyrimidine monomer, respectively. Because of the good availability of cyanuric chloride, the triazine based monomers are especially preferred. Preferably, the synthesis is carried out according to the following reaction scheme:

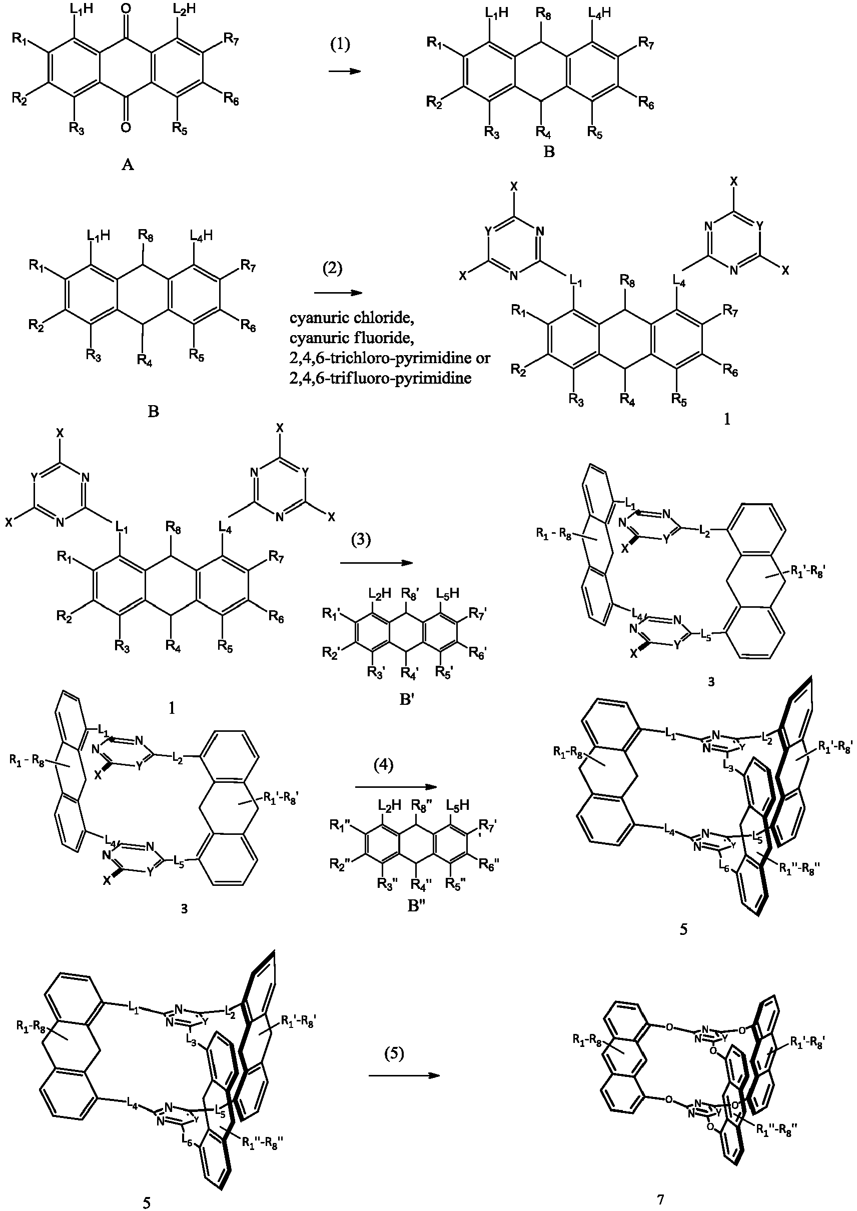

The key step of the synthesis is step (4) in the above reaction scheme, that is, reacting compound of formula 3 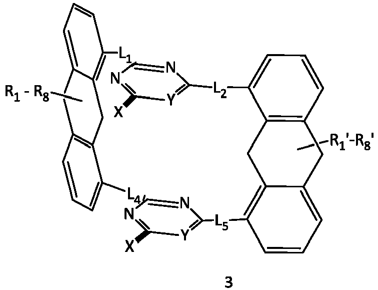 with compound B" 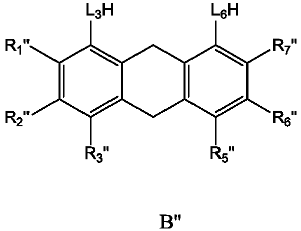 under basic conditions to form the compound of formula 5

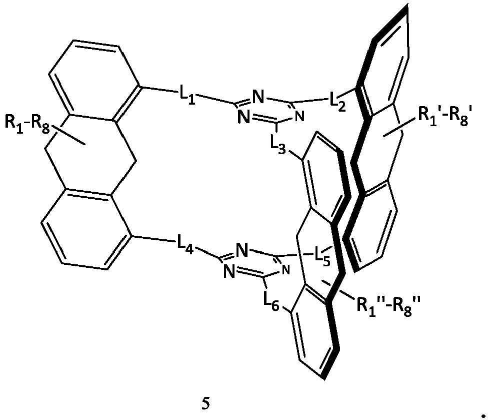

Since the reaction is carried out under basic conditions, it is important that the anthracene derivative is stable under basic conditions, which is, for example, not the case if the 1,8-dihydroxy anthracene is used. Much better results can be obtained by using the reduced analogue of the formula B". The basic conditions are required in order to facilitate the nucleophilic displacement at halogenide atom X in the compound of formula 3. Furthermore, because of the sterically demanding residues in the 4 and 8 positions of compound B" the conditions ensure the symmetric configuration of compound 5 and can have a positive impact on ensuring the symmetric configuration of 7. Preferably, the reaction takes place under high dilution condition in a refluxing organic solvent such as tetrahydrofuran (THF). The basic conditions can be obtained for example by using potassium carbonate or caesium carbonate as base. Compound 5 can be isolated by simple filtration or centrifugation.

Alternatively, the reaction to compound 5 can also be performed in a one-pot reaction, where no workup between the individual substitution steps is required, which is very efficient.

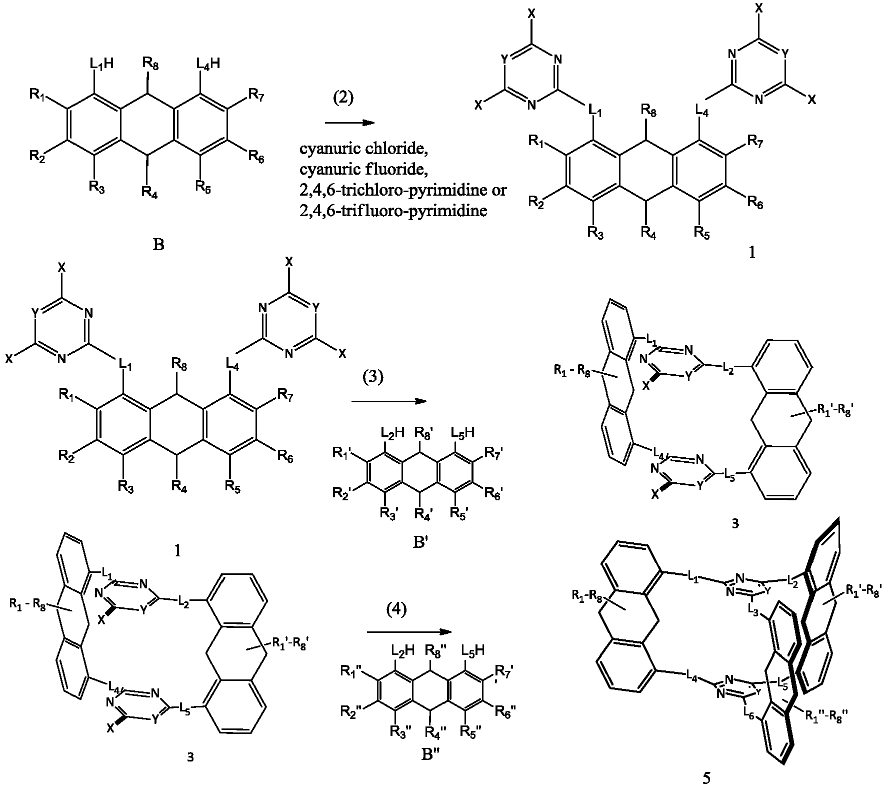

In this alternative, compound B (B', B") is added stepwise to the reaction mixture. The first nucleophilic replacement is carried out using for example DIPEA or caesium carbonate as a base, for the subsequent replacement reactions to compound 3 and compound 5 caesium carbonate or potassium carbonate, preferably caesium carbonate, is used as base. Preferably, caesium carbonate is used with catalytic amounts of 18-crown-6, which increases reaction speed. The reaction can be carried out in suspension leading to high concentrations of compound 5 in the reaction mixture.

Alternatively, the compound of formula 5 may be obtained by the following synthesis: 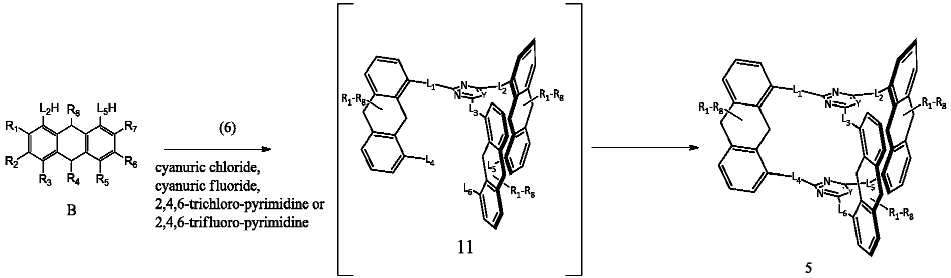 Although the compound of formula 3 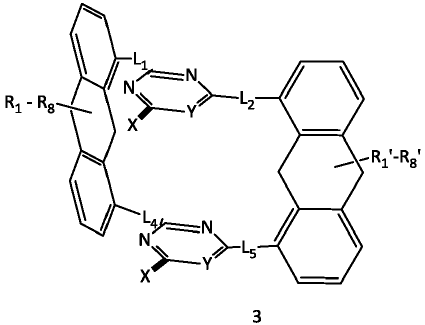 could be obtained differently, it is preferably obtained by reacting compound of formula 1 (step 3 in the above reaction scheme) 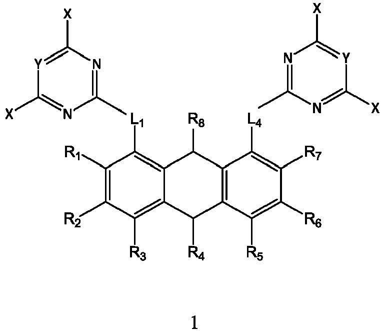 with compound B' 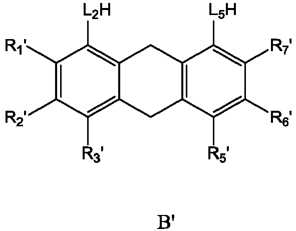 preferably under basic conditions.

Preferably, the reaction is carried out at room temperature. The purification of compound 3 is very easy since it can be isolated by simple filtration.

Preferably, compound of formula 7 is obtained by dehydrogenation compound of formula 5 (step 5 in the above reaction scheme). Good results could be obtained by using 2,3-dichloro-5,6-dicyano-1,4-benzoquinone, chloranil or alpha methyl styrene.

The compound of formula 1 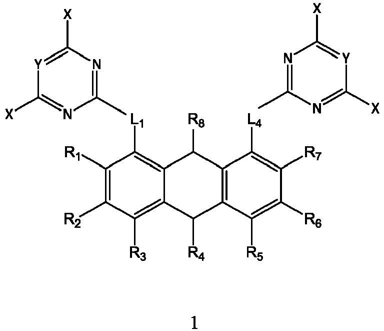 is preferably obtained by reacting compound of formula B 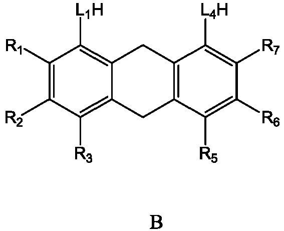 with cyanuric chloride (X is Cl), cyanuric fluoride (X is F), 2,4,6-trichloro-pyrimidine (X is Cl), or 2,4,6-trifluoro-pyrimidine (X is F) (2 in the above reaction scheme).

Another embodiment of the present invention also includes the depolymerization of the polymer according to the present invention. The depolymerization, that is, the conversion of the polymer into the monomer units can be carried out in a controlled manner, which is a highly wanted feature of a polymer under environmental aspects. It can be induced by thermal treatment or by UV light or by pressure or a combination of two or all three treatments. Under the precondition of maintaining the uniaxial or biaxial oriented state of the polymer after depolymerisation, polymerization, as described above, can repeatedly be performed, which enables switching between two different compounds, that is the monomer units and the polymers, with two different property sets.

Yet another embodiment of the present invention also includes patterning, preferably by a photomask, of either the crystals of the monomer unit by UV irradiation with the conditions mentioned above to polymerize specific domains, or of the polymer crystals by specifically depolymerizing certain regions by either UV-light, or by thermal treatment. The patterning can lead to hybrid crystal structures in which both monomer and polymer domains coexist, hereby forming domains of different properties within the crystal.

A further aspect of the present invention relates to the following compounds, which are either monomer units or important intermediate products for the preparation of the monomer units.

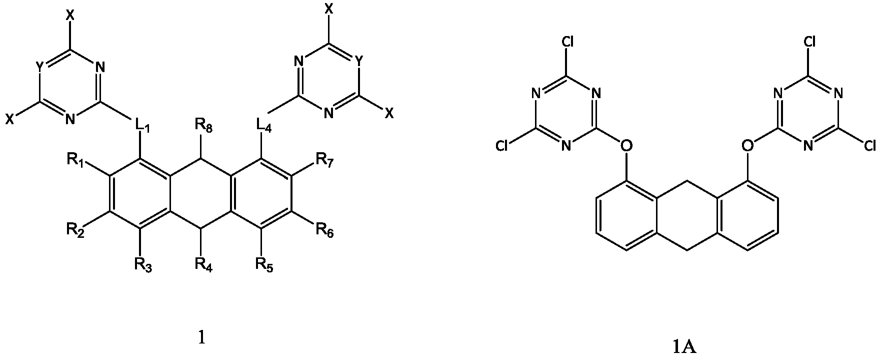

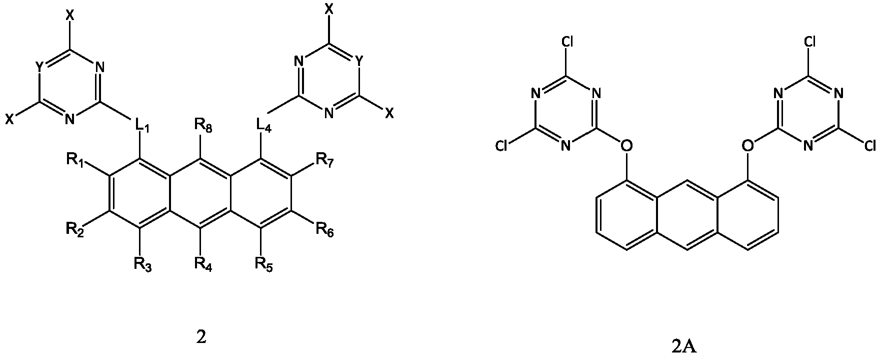

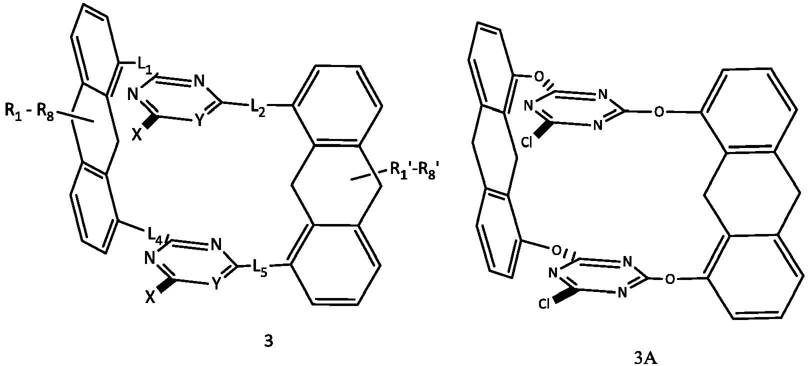

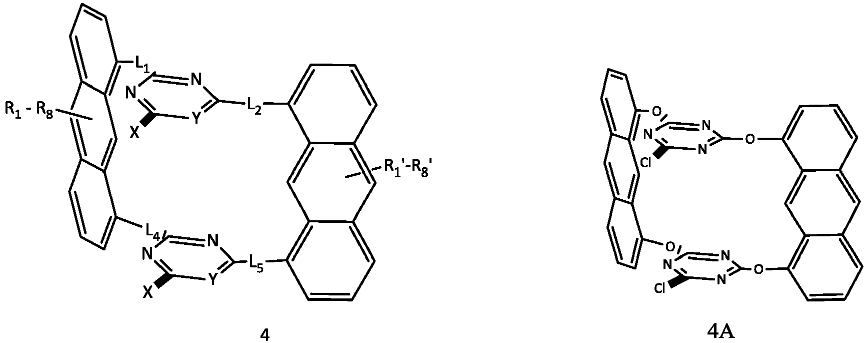

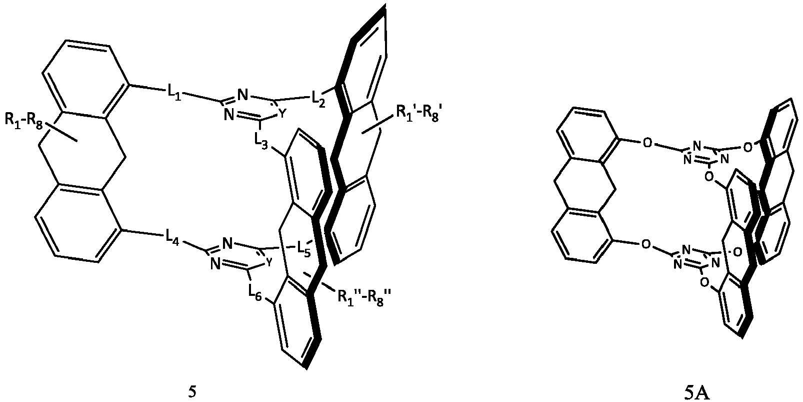

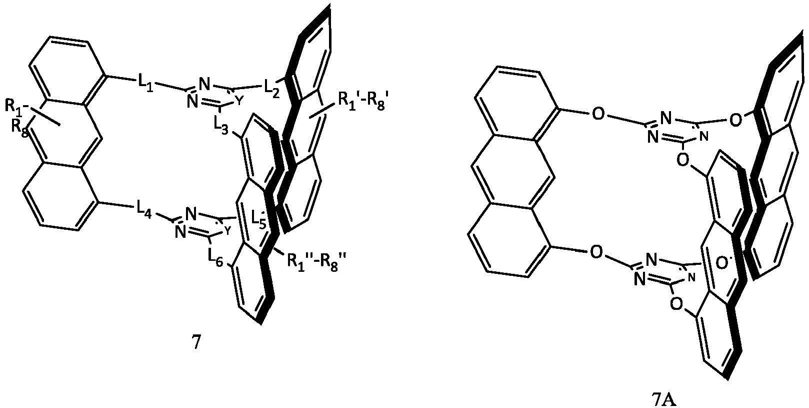

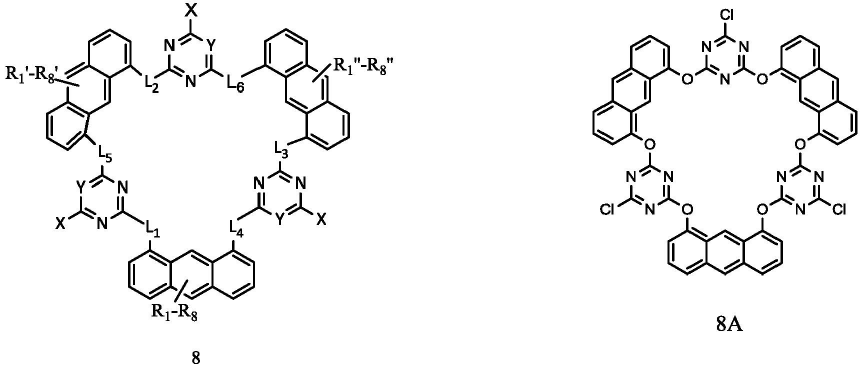

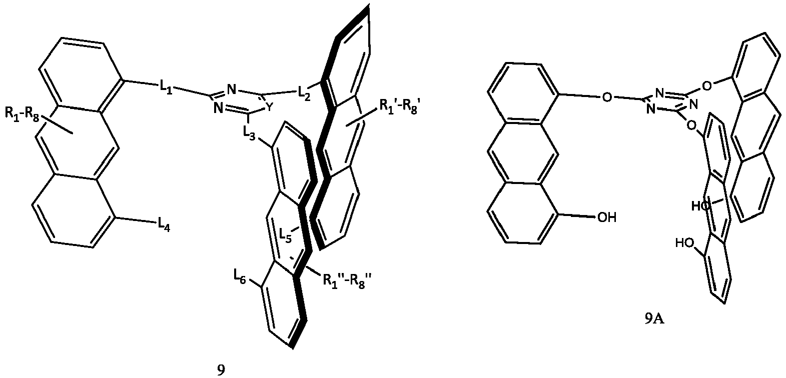

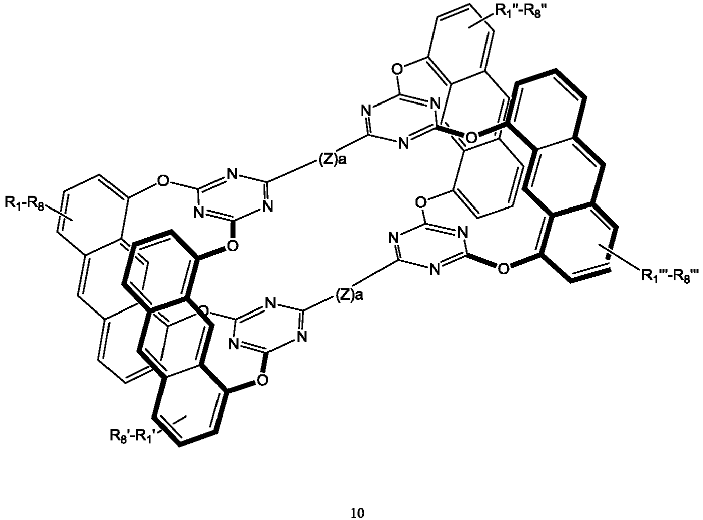

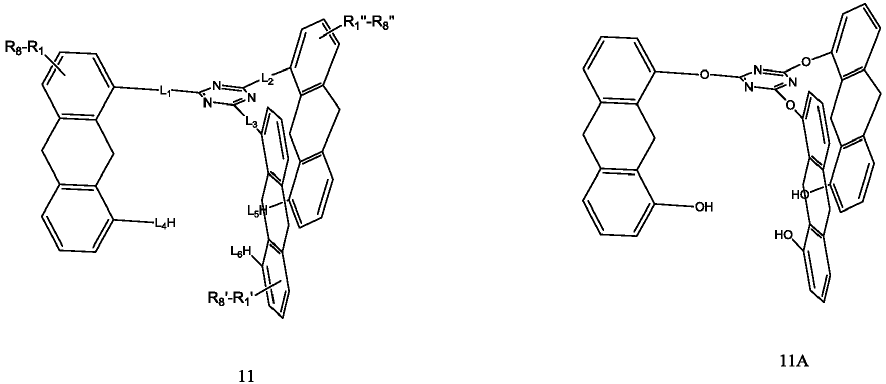

### Figures

- Fig. 2: is a depiction of an X-ray crystal structure of a crystallized form of monomer unit 7a.
- Fig.3: is a depiction of an X-ray crystal structure of a crystallized form of monomer unit 7a with its unit cell from the side.
- Fig 4: is a depiction of the polymerized crystal structure made by monomer unit 7a.
- Fig. 5: is a scanning electron microscope (SEM) image of a thin polymer sheet

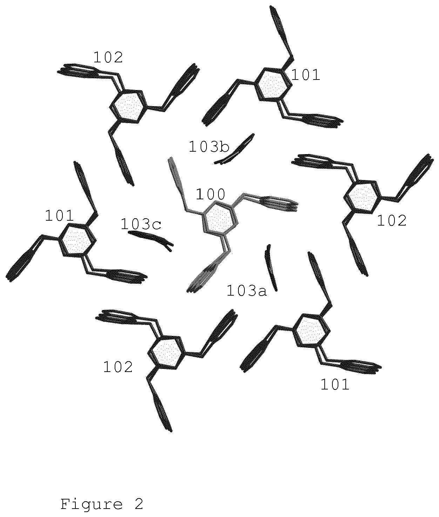

Figure 2 shows a detailed view of the noncentrosymmetric, enantiomorphic crystal structure (space group *R*3 (no. 146)) of the monomer compound 7a, illustrating a specific interaction of monomer unit 7a with the solvent 2-cyanopyridine. It is shown that the solvent molecule and the monomer unit 7a form a network-like arrangement comprising a structural unit. Said structural unit comprises three crystallographically independent 7a molecules a template monomer unit 100, which does not react and two reactive 7a monomer units 101, 102. Around the template monomer unit 100 three 2-cyanopyridine molecules 103a, 103b and 103c are arranged, which are surrounded by reactive monomer units 101, 102. The reactive monomer units 101 and 102 can be polymerized.

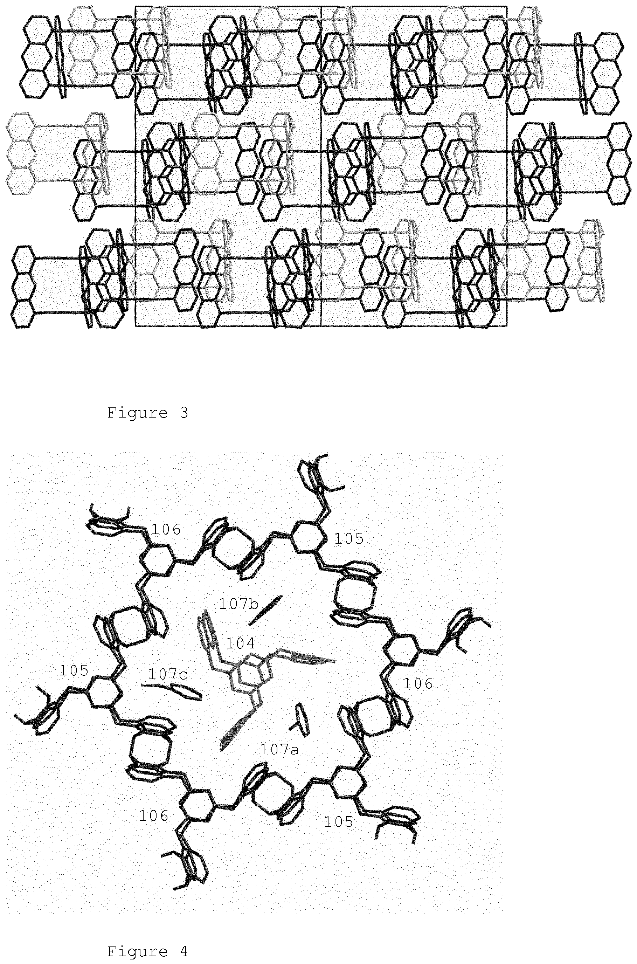

Figure 3 shows the unit cell side view of the crystal structure of monomer compound of formula 7a grown from 2-cyanopyridine. The layered crystal structure is a beneficial precondition for lateral polymerization.

Figure 4 shows the packing of the noncentrosymmetric, enantiomorphic crystal structure (space group P3₁ (no. 144)) of the polymerized crystal. The monomer unit 104 of the compound of formula 7a in the template is not engaged in the polymerization and is tilted, whereas the monomer unit 105 and monomer unit 106 are polymerized, based on the [4+4] photodimerization of the anthracene moieties, resulting in the shown polymeric structure. The 2-cyanopyridine molecules 107a, 107b, 107c adopt oriented positions, in which their nitrile groups face similar directions, herby creating a dipole moment.

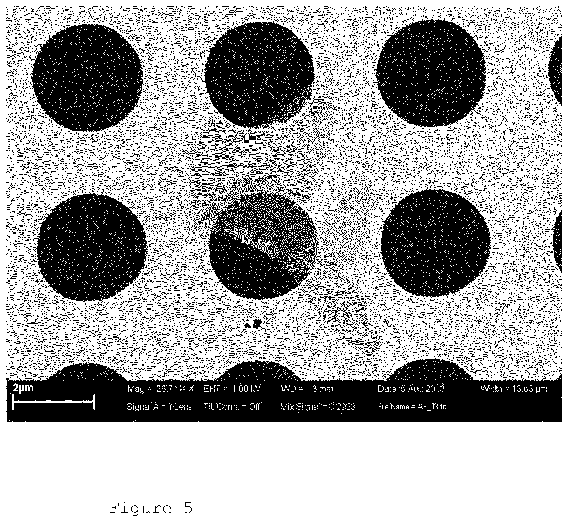

Figure 5 shows an SEM image of a thin polymer sheet obtained through exfoliation deposited on a Quantifoil® TEM grid.

### Examples

NMR spectra were recorded on Bruker AVANCE (¹H: 300 MHz, ¹³C: 75 MHz) at room temperature or on a high resolution Bruker AVANCE spectrometer (¹H: 700 MHz, ¹³C: MHz) using chloroform-d, methanol-d₄, 1,1',2,2'-tetrachloroethane-d₂, DMSO-d₆. Solvent signals were used as internal standard for chemical shift (¹H: δ = 7.26 ppm and ¹³C: δ = 77.16 ppm for chloroform, ¹H: δ = 4.8 ppm and ¹³C: δ = 49.15 ppm for methanol, ¹H: δ = 6.00 ppm and ¹³C: δ = 74.00 ppm for 1,1',2,2'-tetrachloroethane.

The X-ray crystal structure data was collected at 100 K using Mo Kα radiation (0.7107 nm). Solution and refinement were performed using the SHELXS and SHELXL (Sheldrick, 2008).

Infrared spectroscopic analyses were carried out using attenuated total reflection (ATR) - Fourier transform infrared (FTIR) spectrometer (Bruker Optics Alpha system with a built-in diamond ATR). OPUS 6 from Bruker was used as software. The data represent the average of 128 scans in the wavenumber range between 4000 - 400 cm⁻¹ at a resolution of 1.42 cm⁻¹.

Scanning electron microscopy was carried out on a FEG-SEM, Zeiss LEO Gemini 1530, Germany microscope with an in-lens detector using Quantifoil® TEM grids which were placed on a holder (PLANO, G3662). Images were recorded at low beam energy (1 -3 kV) to avoid potential damaging of the thin polymer films. For imaging a chloroform suspension of partially exfoliated crystals and thin polymer sheets was depositited onto a Quantifoil® TEM grid (R2/2, Cu 400 mesh, Quantifoil Micro Tools GmbH, Grosslöbichau, Germany) which was placed carbon side up on a Kimwipe® tissue, so that the solvent would pass through the grid and be absorbed by the tissue. The sample was dried over a few hours.

High resolution mass spectroscopy (HRMS) analyses were performed with spectrometers (MALDI-ICR-FTMS: IonSpec Ultima Instrument, MALDI-TOF: Bruker Ultraflex II). Either 3-hydroxypicolinic acid (3-HPA) or trans-2-[3-(4-tert-butylphenyl)-2-methyl-2-propenylidenemalononitrile (DCTB) was used as matrix in the presence of silver triflate for the latter.

Differential Scanning Calorimetry was performed using a DSC Q1000 (TA Instrument, USA) in N₂-atmosphere with aluminum pans. Sample sizes ranged between 2-4mg. Alternatively, a DSC822 instrument (Mettler Toledo, Greifensee, Switzerland) was used.

### Synthesis of 1,8-bis((4,6-dichloro-1,3,5-triazin-2-yl)oxy)-9,10-dihydroanthracene (1A)

A mixture of 1,8-dihydroxy-9,10-diyhdro anthracene (4.25 g, 20 mmol) and diisopropylamine (DIPEA) (6.204 g, 48 mmol) dissolved in THF (150mL) was added dropwise over 1.5 h to an ice-bath cooled solution of cyanuric chloride (9.61 g, 50 mmol) in THF (300 mL). After 3 hours TLC revealed full consumption of the starting material, the solvent was removed under reduced pressure and the obtained substance was redissolved in chloroform, washed with cold water and dried over MgSO₄. After removal of the chloroform, the substance was washed with cold diethyl ether to give 1A (9.86g, 97 %).
¹H NMR (CDCl₃) δ/ppm: 7.31 (m, 4H) ; 7.00 (m, 2H) ; 4.14 (s, 2H) ; 3.65 (s, 2H).
¹³C NMR (CDCl₃) δ/ppm: 173.5; 171.1; 148.87; 138.46; 127.7; 126.6; 126.45; 119.1; 35.6; 23.3.
HRMS (ESI/MALDI-FTICR, DCTB) calcd for C₂₀H₁₁Cl₄N₆O₂⁺ 506.9692 [MH]⁺, found 506.9689.
Anal. Calcd for C₂₀H₁₀Cl₄N₆O₂: C, 47.27; H, 1.98; Cl, 27.91 N, 16.54; Found: C, 47.22; H, 2.01; Cl, 28.05; N, 16.66.

### Synthesis of 1,8-bis((4,6-dichloro-1,3,5-triazin-2-yl)oxy)anthracene (2A)

Compound 1A (3.5g, 6.89mmol) was dissolved in 1,2 dichloroethane and purged with argon for 30 minutes. To this mixture 2,3-dichloro-5,6-dicyano-1,4-benzoquinone (2.2 g, 9.7 mmol) was added and the mixture refluxed for 3 hours. After reaction completion the solvent was removed under reduced pressure. The resulting residue was washed various times with cold methanol to give 2A (2.5g, yield: 71%).
¹H NMR (CDCl₃) δ/ppm: 8.62 (s, 1H); 8.40 (s, 1H); 8.03 (d, 2H); 7.55 (dd, 2H); 7.33 (d, 2H).
¹³C NMR (CDCl₃) δ/ppm: 173.5; 171.8; 147.4; 133.2; 128; 127.7; 125.4; 125.3; 117.6; 113.5.

### Synthesis of 3A:

1,8-dihydroxy-9,10-diyhdro was added to a flask charged with DIPEA (3.102 g, 24 mmol) and chloroform (2.6 L). The reaction mixture was stirred and slightly warmed until a clear solution was obtained. The solution was left to cool back to room temperature. Compound 1A dissolved in chloroform (400 mL) was added to this mixture at once. The resulting mixture was then stirred at room temperature over 3 days. After TLC indicated complete consumption of the starting materials, the solvent was removed under reduced pressure and the precipitate was collected by filtration, washed with cold dichloromethane and dried to give 3A as a white substance (5.02 g, 77.5 %).
¹H NMR (C₂D₂Cl₄) δ/ppm: 7.39-7.32 (m, 8H) ; 7.07 (d, 4H) ; 4.2 (s, 4H); 3.67-3.36 (m, 4H).
¹³C NMR (C₂D₂Cl₄) δ/ppm: 173.7; 171.6; 148.5; 137.4; 127.8; 126.4; 126.1; 118.9; 34.8; 23.3.
HRMS (ESI/MALDI-FTICR, DCTB) calcd for C₃₄H₂₁Cl₂N₆O₄⁺ 647.0996 [MH]⁺; found 647.0995.
Anal. Calcd for C₃₄H₂₀Cl₂N₆O₄: C, 63.07; H, 3.11; N, 12.98; O, 9.88. Found: C, 62.96; H, 3.12; N, 12.94, O, 10.07.

### Synthesis of 4A:

Compound 3A (324 mg, 0.5 mmol) was dissolved in 800 mL of tetrachloroethane. The solution was purged with argon for 30 min. 2,3-dichloro-5,6-dicyano-1,4-benzoquinone (340 mg, 1.5 mmol) was added thereto and the reaction mixture is heated to reflux for 4 hours. The solvent was removed under reduced pressure and the obtained substance was washed cold with methanol and dichloromethane to give 4A (450 mg, 70%).
¹H NMR (C₂D₂Cl₄) δ/ppm: 8.69 (s, 2H); 8.09 (d, 4H); 8.04 (s, 2H); 7.6(dd, 4H); 7.36(d, 4H).

### Synthesis of 5A (alternative 1):

To a refluxing solution of 1,8-dihydroxy-9,10-diyhdro (1.06 g, 5 mmol) and K₂CO₃ (1.73g, 12.5 mmol) in THF (2250 mL) was added 1,8-dihydroxy-9,10-diyhdro anthracene (3.24 g, 5 mmol) in THF (750 mL) at once. The resulting mixture was stirred under reflux over 5 days, after which the precipitate was collected by centrifugation, washed various times with a cold THF/H₂O mixture (3:1) and methanol and then dried to give 5A as a white crystalline substance (3.18 g, 81 %).
¹H NMR (C₂D₂Cl₄) δ/ppm: 7.46-7.38 (m, 12H, H-2, H-3); 7.08 (d, J = 7.2 Hz, 6H, H-1); 4.34-4.12 (m, 6H, H-5); 3.79-3.72 (m, 3H, H-4); 3.42-3.33 (m, 3H, H-4).
¹³C NMR (C₂D₂Cl₄) δ/ppm: 173.1; 148.7; 137.7; 128.0; 126.8; 126.6; 118.9; 35.2; 23.6.
HRMS (ESI/MALDI-FTICR, DCTB) calcd for C₄₈H₃₁N₆O₆⁺ 787.2300 [MH]⁺; found 783.2301.

### Synthesis of 5A (alternative 2):

In an alternative approach, 5A, can be synthesized in one-pot at high concentrations.

THF (200 mL) was added to a 3-necked 500 mL round-bottom flask and cooled to 10 °C. Sequentially, cyanuric chloride (7.0445 g, 0.0382 mol, 2 eq), 1,8-dihydroxy-9,10-dihydro-anthracene (4.0538 g, 0.0191 mol, 1 eq), DIPEA (3.7027 g, 0.0286mol, 1.5 eq), cesium carbonate (15.557 g, 0.0477mol) and 18-crown-6 (1.2621 g, 0.00478mol, 0.25 eq) was transferred and the reaction progress monitored by TLC. In less than 1 hour, full reaction to 1A was reached. 1,8-dihydroxy-9,10-dihydro-anthracene (4.0538 g, 0.0191 mol, 1 eq) and cesium carbonate (15.557 g, 0.0477mol) was then added and the reaction mixture let come to room temperature. TLC indicated full conversion to 3A after a few hours. 1,8-dihydroxy-9,10-dihydro-anthracene (4.0538 g, 0.0191 mol, 1 eq) was then added and the reaction heated to reflux. After a few hours, TLC showed full conversion to 5A. The reaction mixture was allowed to cool to room temperature. Subsequently, a water/ethanol mixture (2:1) was added (750 mL) and the resulting mixture stirred, after which the mixture was filtered, and thoroughly washed with additional water and ethanol. The precipitate was dried. Yield: 13.2 g (88.0 %)

### Synthesis of compound 7a (alternative 1)

Compound 5A (500 mg, 0.636 mmol, 1 eq) was dissolved in 20 mL of argon-purged diphenylether and heated to reflux. Chloranil (545.4 mg, 2.218 mmol, 3.5 eq) was added thereto and 5 minutes later the reaction mixture was brought to room temperature. The precipitate was collected and washed with ethanol. Yield: 468.8 mg (93.9%).
¹H-NMR spectroscopy could not be performed due to the insolubility of 7a also in potent deuterated solvents.
HRMS (ESI/MALDI-FTICR, DCTB) calcd for C₄₈H₂₄N₆O₆ 780.1752⁺ [M]⁺; found 780.1751.

### Synthesis of compound 7a (alternative 2)

Compound 5a (30 mg, 0.038 mmol) was added to 15 mL diphenyl ether. It was heated to reflux to form a clear yellow solution. 4 mL of α-methylstyrene was added thereto and the reaction mixture was refluxed over 3 days at 260°C.

After the reaction mixture was cooled to room temperature, a crystalline precipitate formed. Yield: 21mg (71%).
¹H-NMR spectroscopy could not be performed due to the insolubility of 7a also in potent deuterated solvents.
HRMS (ESI/MALDI-FTICR, DCTB) calcd for C₄₈H₂₄N₆O₆ 780.1752⁺ [M]⁺; found 780.1751.

### Synthesis of 8A

To a suspension of NaH (50 mg, 2.1 mmol) in 25mL of dry, ice cold, under an argon atmosphere in THF was added 1,8-dihydroxy anthracene in dry, oxygen-free THF (5 mL) and stirred for 30 minutes which the mixture was carefully warmed until no effervescence was visible. The suspension was then diluted by adding dry, oxygen-free THF (320 mL) under argon. Cyanuric chloride (129 mg, 0.7 mol) dissolved in THF (5 mL) was then added at once and the entire mixture stirred at rt overnight. The solvent was removed under reduced pressure and the obtained substance was dissolved in chloroform, washed with water and dried over MgSO₄. Recrystallization from CHCl₃/hexane gave yellow crystals (51 mg, 15 %).
HRMS (MALDI-) calcd for C₅₁H₂₄Cl₃N₉O₆ [M]⁺ 963.0915, found 963.0915.
¹H NMR (CDCl₃) δ/ppm: 8.24 (s, 3H), 8.16(s, 3H), 7.65 (d, J 6H), 7.18 (dd, 6H) 6.97 (d, 6H).
¹³C NMR (CDCl₃) δ/ppm: 174.6; 172.5; 147.2; 132.3; 126.6; 126.3; 124.6; 124.4; 116.6; 114.

### Synthesis of 11

Into a Schlenktube (10 mL), 1,8-dihydroxy-9,10-diyhdro (191 mg 0.9 mmol) was added and dissolved in dry THF (1mL). The mixture was cooled to 0°C after which BuLi (0.56 mL, 0.9 mmol) was added and stirred for 10 min. To this mixture cyanuric chloride (55mg, 0.3mmol) dissolved in THF (0.5mL) was added over 1 hour, stirred overnight letting the reaction slowly come to rt. The reaction mixture was precipitated in methanol to remove oligomeric and polymeric side products. After filtration, and removal of solvent under reduced pressure the obtained substance was subjected to column chromatography (EtOAc 1: Hexane 2) to give **11.** (40 mg, 19%).
¹H NMR (3MeOD:1CDCl₃) δ/ppm: 6.98 (d, J = 7.7Hz, 3H), 6.93-6.86 (m, 6H) ; 6.75 (d, 3H) ; 6.67 (d, 3H) ; 6.55 (d, 3H) ; 3.72 (s, 2H) ; 3.53 (s, 2H).
¹³C NMR (3MeOD:1CDCl₃) δ/ppm: 173.3; 153.3; 148.9; 138.1; 136.8; 128.3; 126.0; 125.9; 124.6; 121.1; 118.2; 118.1; 111.9; 34.8; 21.8.
HRMS (ESI/MALDI-FTICR, DCTB) calcd for C₄₅H₃₃N₃O₆; 734.2262, [M+Na]⁺ found 734.2261.

### Crystallization of Compound 7a

### Method 1:

Round or hexagonal platelets with diameters of ca. 20 µm were obtained by cooling down a hot solution (212°C) of 7a to room temperature in an hot oil bath over 2.5 hours (conc. 6-7 mg/ml)

### Method 2:

Cylinder or prism-shaped crystals with diameters of 5-10 µm were obtained by cooling down a hot solution of 7a in 2-cyanopyridine at 212°C (6-7 mg/mL) to room temperature by letting the hot vial air cool on the work bench for ca. 30 minutes.

### Method 3:

Crystals of larger dimensions (diameters ≥ 200 µm), were obtained by cooling down a vial containing a solution of 7a in 2-cyanopyridine at 212°C (6-7 mg/ml) to 50°C over 48 hours using a heating plate which was connected to a PID controller for gradient cooling.

### Method 4:

C60 fullerenes/compound 7a cocrystals were obtained by mixing a solution of Fullerene C60 (20 mg) in 5 mL of o-dichlorobenzene and a refluxing solution of M3O (43 mg) in 2-cyanopyridine (10mL). Immediate crystallization into hexagonal crystals occured. The combined mixture were then filtered and the obtained crystals washed with methanol.

### Method 5:

C70 fullerenes/compound 7a cocrystals were obtained by mixing a solution of Fullerene C70 (20mg) in 5 mL of o-dichlorobenzene and a refluxing solution of M3O (37mg) in 2-cyanopyridine (10mL). Immediate crystallization into hexagonal crystals occurred. The combined hot mixture were filtered and the obtained crystals washed with methanol.

### Photopolymerization of crystals of compound 7a grown from 2-cyanopyridine:

### Crystal transformation on a small scale:

Single crystals of compound **3** from 2-cyanopyridine were deposited on a glass slide in a perfluoropolyalkylether. The glass slide was placed onto the photoreactor with a nominal power dissipation of 16 x 2.8 W (high power LEDs) = 44.8 W (ca. 30 Lumen per LED at ca. *I* = 400 mA), the crystals were irradiated for 24 hours at a wavelength of λ = 465 nm (XRD).

### Crystal transformation on a larger scale in dispersion, "batch procedure":

A Schlenk flask was charged with crystals of 7a (1 g, 1.28 mmol; 5-30 µm) grown from 2-cyanopyridine. They were dispersed in 15 mL of argon purged methanol/water mixture (2:1). The mixture was stirred under an argon environment and irradiated with a custom-made photo-reactor (465 nm), which was mounted onto a magnetic stirrer. The photo reactor had a nominal power dissipation of 16 x 2.8 W (high power LEDs) = 44.8 W (ca. 30 Lumen per LED at ca. *I* = 400 mA). Irradiation for 8 hours afforded the polymeric product.

### Thermally induced depolymerization crystalline state:

Two single crystals (sizes ca. 200µm) which were analysed with respect to their polymeric nature by single crystals X-ray structure analysis were deposited on a microscope glass slide and heated in an oven (Heraeus Vaccutherm 6000) at 180°C over a time period of three days to up to three weeks. The resulting crystals were subsequently analysed by XRD. For the crystals heated for three days, monomer concentrations of 59% and 77% were determined. Crystals heated for three weeks consisted of monomer exclusively.

### Alternatively, a sample with polymerized crystals (5-30 µm) grown form 2-cyanopyridine was exposed to a heating cycle using differential scanning calorimentry (DSC) to 275°C (10°C/min) and subsequently cooled down (10°C/min) after which IR revealed full depolymerization to monomer crystals.

### Reversible polymerization/depolymerization of 7a:

A glass vial was charged with crystals of 7a (50 mg, 0.4 mmol; 5-30 µm) grown from 2-cyanopyridine was irradiated for 5 hours under argon using a custom-made photo-reactor (465 nm) a nominal power dissipation of 16 x 2.8 W (high power LEDs) = 44.8 W (ca. 30 Lumen per LED at ca. *I* = 400 mA). Polymerization was confirmed by IR spectroscopy. The polymerized samples where then heated to 200°C in oven (Heraeus Vaccutherm 6000) for 4 days. Full depolymerization could be confirmed by IR spectroscopy. Subsequent irradiation at 465 nm let to polymerization, as was determined once more by IR spectroscopy.

### Exfoliation

Remove solvent from crystals: Irradiated crystals were dispersed in a small glass vial containing ca. 2 mL of heated NMP (N-methyl-2-pyrrolidone) (50°C). This mixture was stirred at 80 rpm over 2-3 weeks. The obtained suspension of partially exfoliated crystals and thin polymer sheets was centrifuged in an Eppendorf vial. The NMP supernatant was removed and the solids were washed with methanol and centrifuged. Subsequently, methanol was removed and chloroform added to form a suspension of thin sheets and partially exfoliated crystals.

Exfolitation in TFA: Polymerized crystals (20-30um) were dispersed in a small glass vial containing ca. 2 mL of trifluoro acetic acid. This mixture was stirred at 80 rpm over 2-3 weeks. The obtained suspension of partially exfoliated crystals and thin polymer sheets was diluted with dichloromethane and subsequently washed with water. Extraction of the organic phase led to a suspension of partially exfoliated crystals and thin polymer sheets.

Micromechanical exfoliation: Polymerized crystals (20-30um) were sprinkled onto adhesive tape, e.g. 3M Magic Tape, Nitto SPV 224P tape. The tape was repetitively folded onto itself and peeled apart.

## Claims

1. Polymer comprising as monomer unit one or more compounds selected from the group consisting of 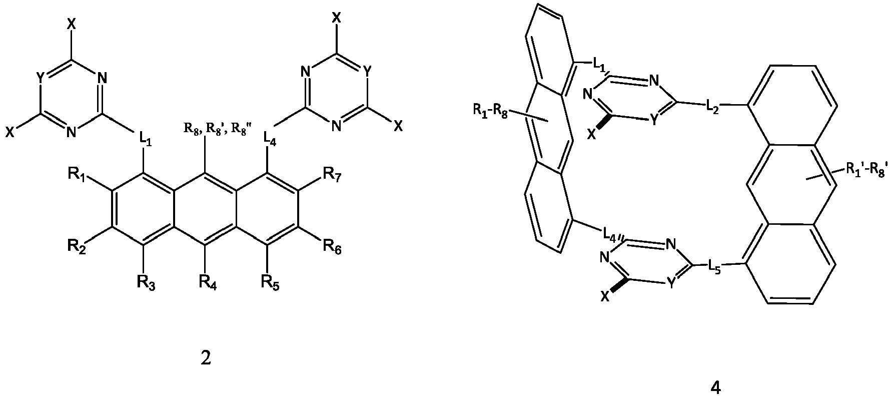 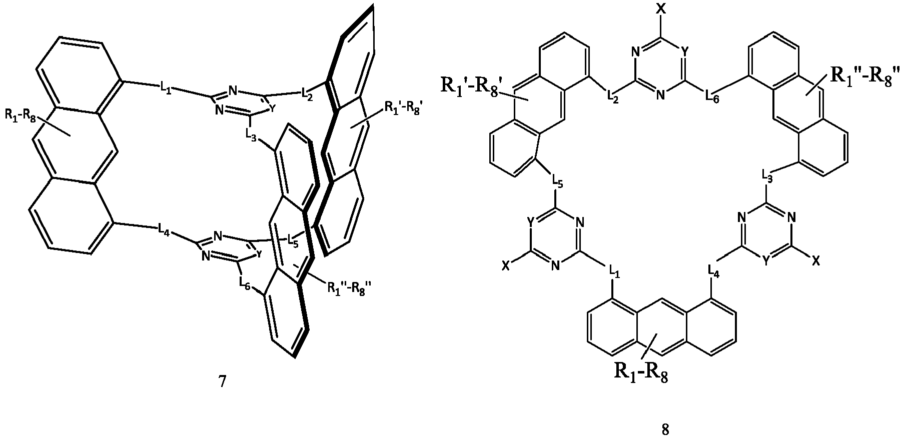 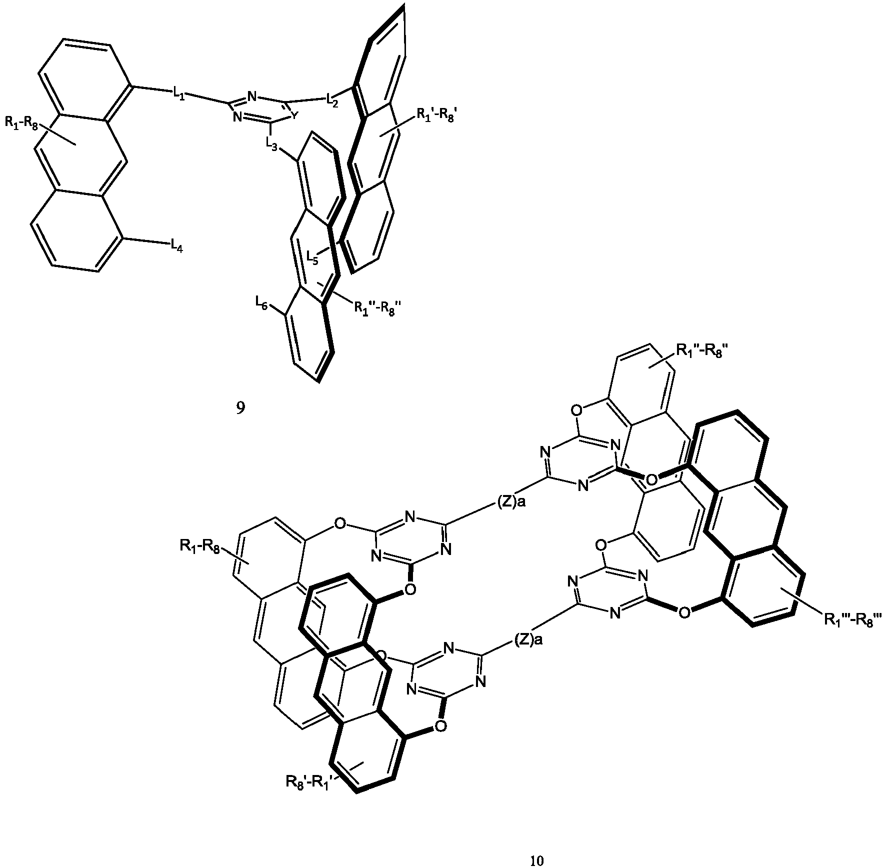 wherein the residues R₁ to R₈ of the first anthracene moiety, R₁' to R₈' of the second anthracene moiety, R₁" to R₈" of the third anthracene moiety and R₁"' to R₈'" of the forth anthracene moiety in formulas 4, 7, 8, 9 and 10 are arranged as follows 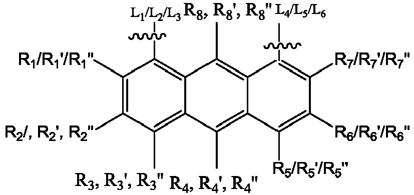 and in formulas 2, 4, 7, 8, 9 and 10 R₁, R₁', R₁", R₁"', R₂, R₂', R₂", R₂'", R₃, R₃', R₃", R₃"', R₄, R₄', R₄", R₄"', R₅, R₅', R₅", R₅"' R₆, R₆', R₆", R₆"', R₇, R₇', R₇", R₇"', R₈, R₈', R₈" and R₈'" are independently from each other hydrogen, deuterium or a linear or branched alkyl group having 1 to 8 carbon atoms, preferably selected from the group of methyl, ethyl or propyl,
each of R₁ and R₂, R₁' and R₂', R₁" and R₂", R₁'" and R₂"', R₂ and R₃, R₂' and R₃', R₂" and R₃", R₂"' and R₃"', R₅ and R₆, R₅' and R_{6'}, R₅" and R₆", R₅"' and R₆"', R₆ and R₇, R₆' and R₇', R₆" and R₇", R₆'" and R₇'" may form independently from each other a six-membered aromatic ring which may optionally be substituted with a linear or branched alkyl group having 1 to 16 carbon atom, preferably selected from the group of methyl, ethyl or propyl residues, and
L₁, L₂, L₃, L₄, L₅ and L₆ are independently from each other oxygen, sulphur, acetylene, or NR₉, and R₉ is hydrogen or methyl, and
X is either chlorine or fluorine,
Y is nitrogen or CH, preferably nitrogen, and
Z is NH, O, S, alkyne, alkylene, arylene, heteroarylene, oligo (ethylene glycol), and (a) is 0 or 1.

2. Polymer comprising as monomer unit a compound of formula 7, preferably a compound of formula 7a 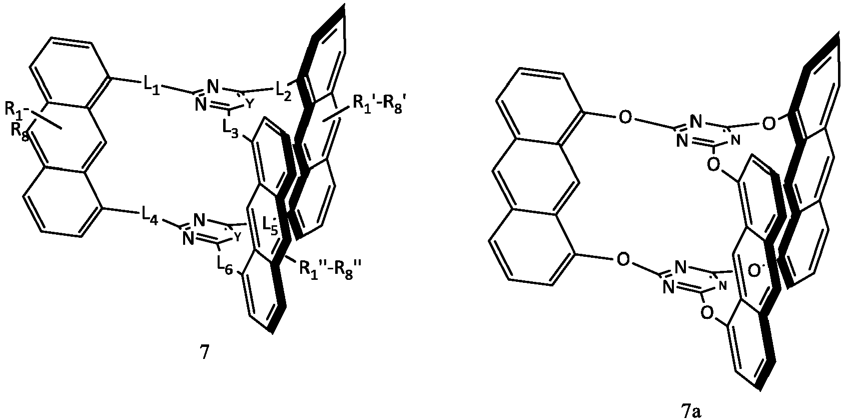 and all residues have the same definition as in claim 1.

3. Polymer according to any of the preceding claims, wherein said polymer is a homopolymer.

4. Polymer according to claim 1 to 2, wherein said polymer is a copolymer, comprising a first monomer unit and a second monomer unit, wherein
the first monomer unit is selected from a compound of formula 2, 4, 7, 8, 9 and 10, preferably compound 7a and
the second monomer unit is either
(i) selected from a compound of formula 2, 4, 7, 8, 9 and 10 but is different from the first monomer unit; or
(ii) is a compound having the same formula as the first monomer unit, but at least one of the residues R₁, R₁', R₁", R₁"', R₂, R₂', R₂", R₂'", R₃, R₃', R₃", R₃"', R₄, R₄', R₄", R₄'", R₅, R₅', R₅", R₅"', R₆, R₆', R₆", R₆"', R₇, R₇', R₇", R₇"', R_{8,} R₈', R₈", R₈'", L₁, L₂, L₃, L₄, L₅, L₆, X or Y is different; or
(iii) a monofunctional or a bifunctional nucleophilic compound; or
(iv) a dienophile, preferably a fullerene, most preferably a C60 fullerene or a C70 fullerene.

5. Polymer according to claim 1 to 4, **characterized in that** the polymer is in a crystalline state or comprises a hybrid crystal structure, comprising monomer unit domains and polymer unit domains.

6. Blend comprising the polymer according to any of the preceding claims and a thermoplastic polymer selected from the group consisting of polyamides, polyesters, polypropylene, polyethylene, polyarylene sulfides, polyarylene oxides, polyimides, polyetherketones, polyetheretherketones, polyoxymethylenes and polyetherimides, polyurethanes, rubbers, polycyanurates, polyaramids and any copolymers thereof or mixtures thereof.

7. Method for preparing a polymer according to claims 1 to 5
a) by irradiating with ultraviolet radiation or
b) by thermally induced polymerization,
c) by pressure induced polymerization
whereby the monomer units are in an uniaxial or biaxial oriented state.

8. Method according to claim 7, wherein before polymerizing the monomer units, the monomer units are oriented by forming crystals or co-crystals from a solvent or a solvent mixture.

9. Method according to claim 7 or 8, wherein, before polymerizing the monomer units, the monomer units are oriented by
a. by forming crystals from a solvent or a solvent mixture
b. by forming crystals from a solvent or a solvent mixture in an electric field
c. by forming co-crystals from a solvent or a solvent mixture
d. by forming co-crystals from a solvent or a solvent mixture in an electric field
e. forming a film at a gas-liquid interface, or
f. forming a film from a solvent or solvent mixture on a solid substrate, or
g. subliming the monomer units to form crystals, or
h. by vapour deposition of the monomer units onto a substrate to form an oriented layer, or
i. subliming the monomer units and beam them onto a substrate forming an oriented layer

10. Method according to claims 7 to 9, wherein the polymer is post-modified by functionalization of the polymer, preferably by alkylating one or more nitrogen atoms in the triazine cores or by hydrogenolysis.

11. Use of the polymer according to claims 1 to 5 or the polymer blend according to claim 6 as optoelectronic devices, light emitting diodes, laser diodes, organic transistors, capacitors, sensors, supercapacitors, dielectrics, high-k dielectrics, holographic devices, energy storage applications, batteries, photovoltaic devices, photonic crystal devices, magnetic devices, ferroelectric devices, piezoelectric devices, pyroelectric devices transparent conductive coatings, nucleating agents, clarifying agents, nonlinear optical devices, wear resistant coatings, lubricating coatings, protein repellent coatings, catalyst supports, scratch resistance coatings, chiral surfaces or as organic superconductors, photocatalysis, data storage applications, packaging, membranes, gas separation membranes or desalination membranes.

12. Method for processing the polymer according to claims 1 to 5 by spin coating, drop casting, dip coating, laminating, spray coating, melt extrusion, reaction injection molding or exfoliation.

13. Method for preparing a compound of formula 7 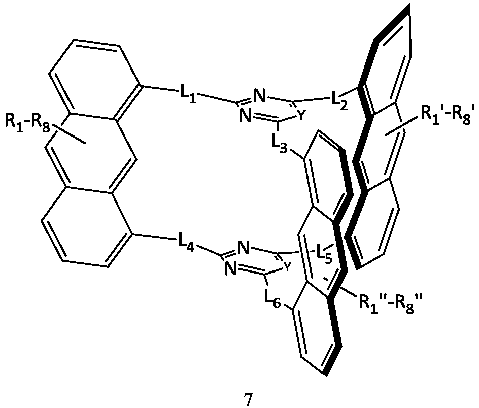 wherein all residues have the same definition as in claim 1,
**characterized in that** it comprises the step of reacting compound of formula 3 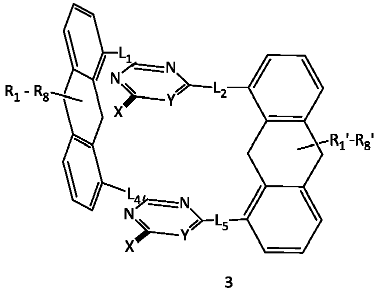 with compound B" 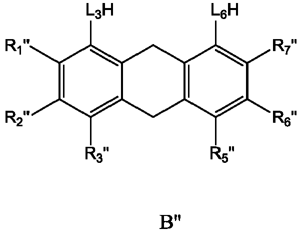 under basic conditions to form the compound of formula 5

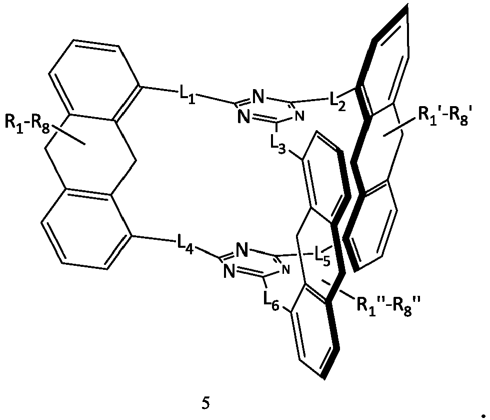

14. Method according to claim 13, wherein compound 5 is formed in a one-pot reaction by reacting 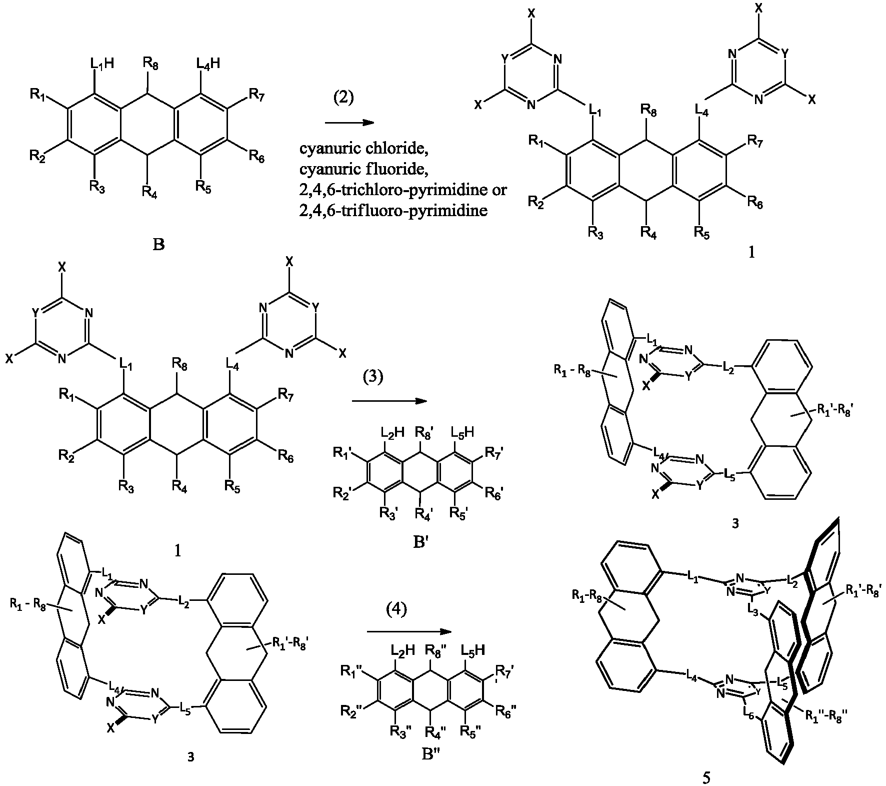 wherein all residues have the same definition as in claim 1,
**characterized in that** it is carried out in the presence of a base, preferably using caesium carbonate.

15. Method according to claim 13, wherein compound of formula 3 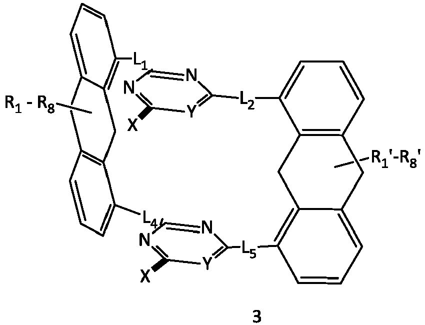 wherein all residues have the same definition as in claim 1,
is obtained by reacting compound of formula 1 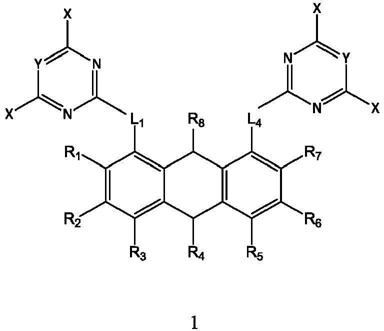 with compound B'

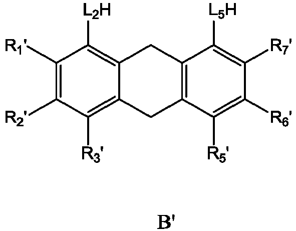

16. Monomer units of formula 2, 4, 7, 8, 9, 10 in a crystalline state, optionally having domain formation with inverted polar axis.

17. Compound of formula 7, preferably compound of formula 7a, in a crystalline state.

18. A method of
inducing depolymerization of the polymer according to claim 1 to 5, preferably by
i. thermal treatment and/or
ii. by UV light and/or
iii. by pressure

19. Compound selected from the group of 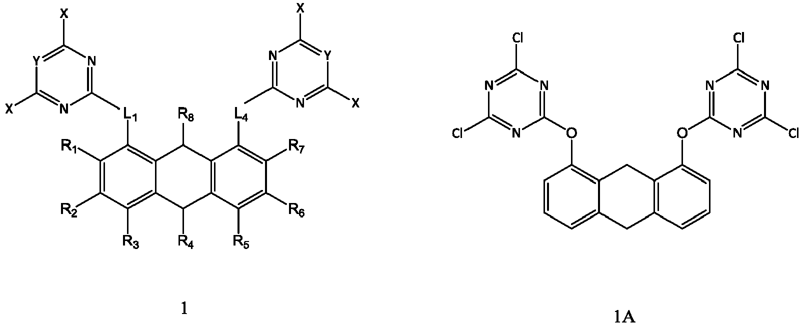 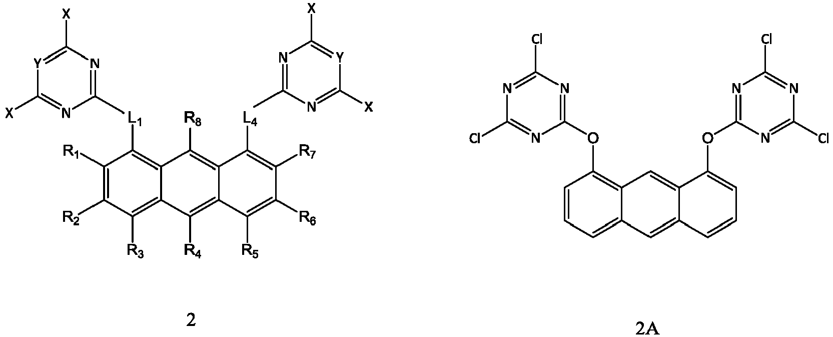 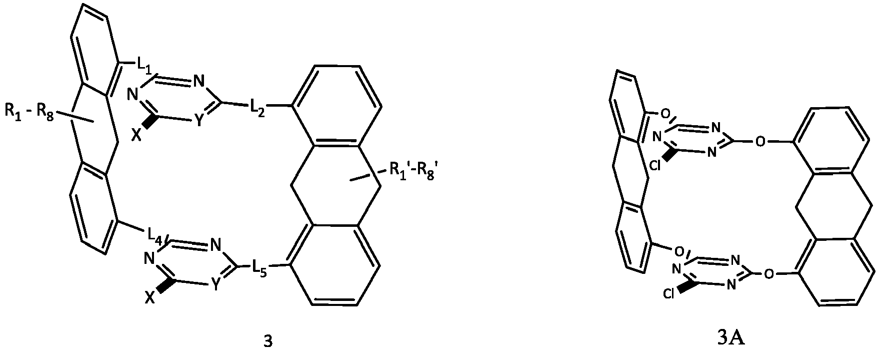 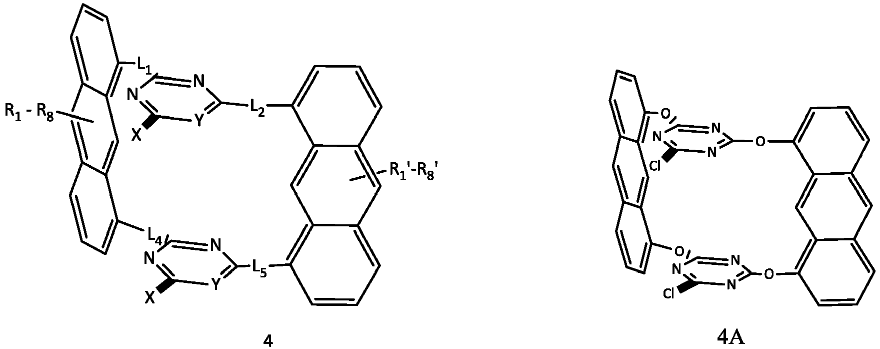 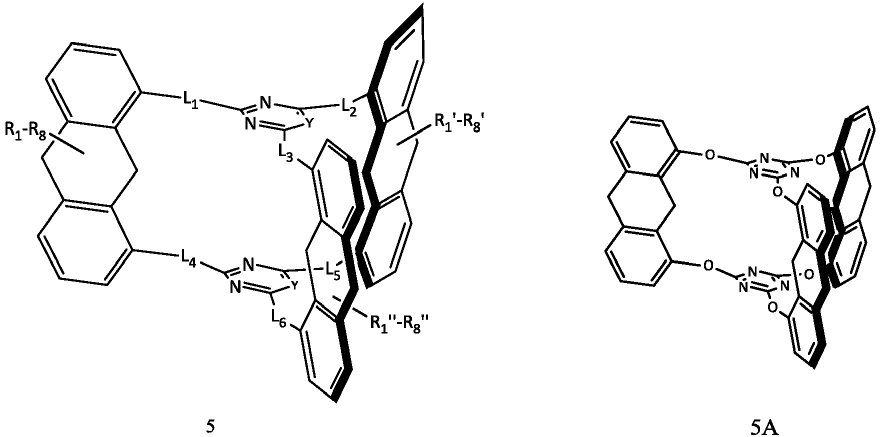 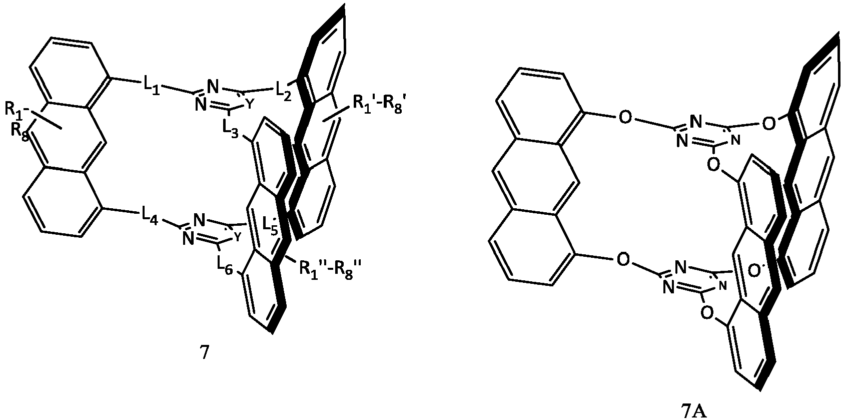 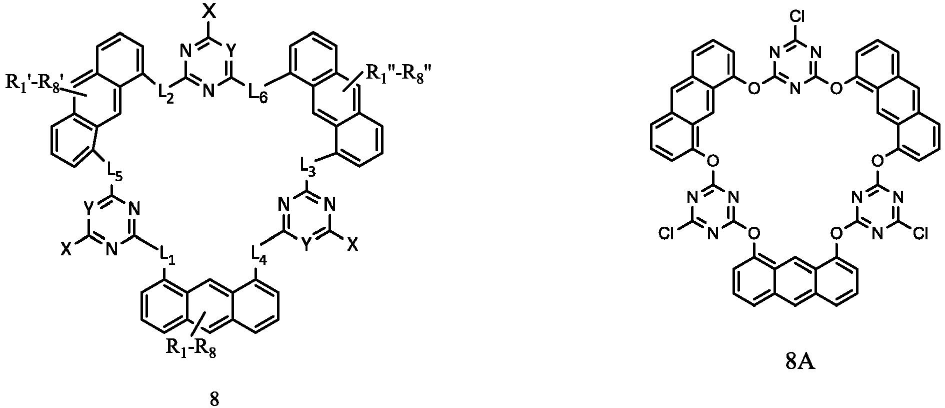 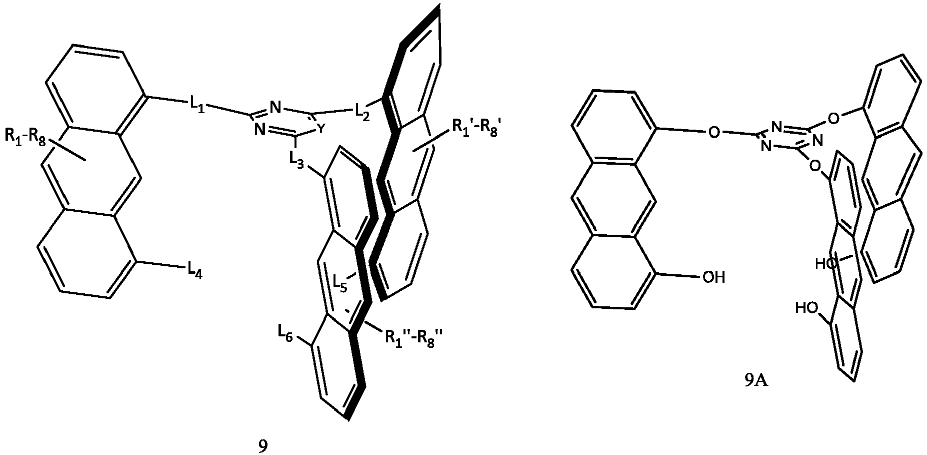 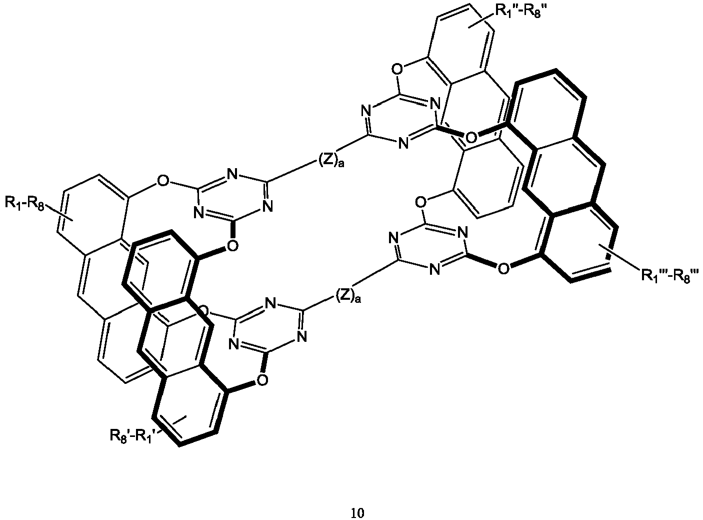 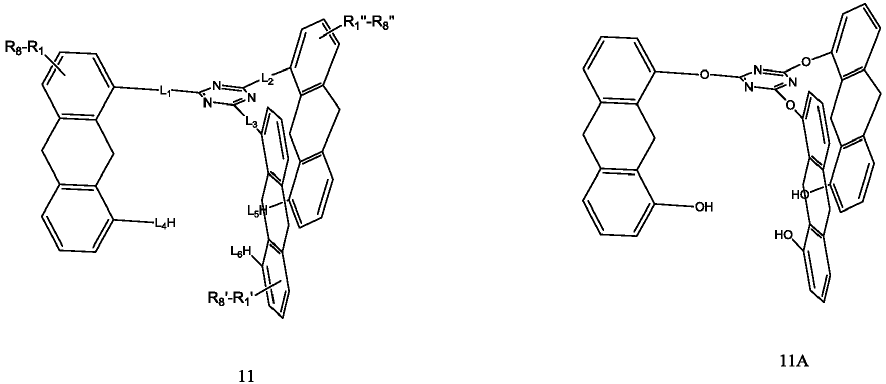 and all residues have the same definition as in claim 1.

## Patentansprüche

1. Polymer enthaltend als Monomereinheit eine oder mehrere Verbindungen ausgewählt aus der Gruppe bestehend aus 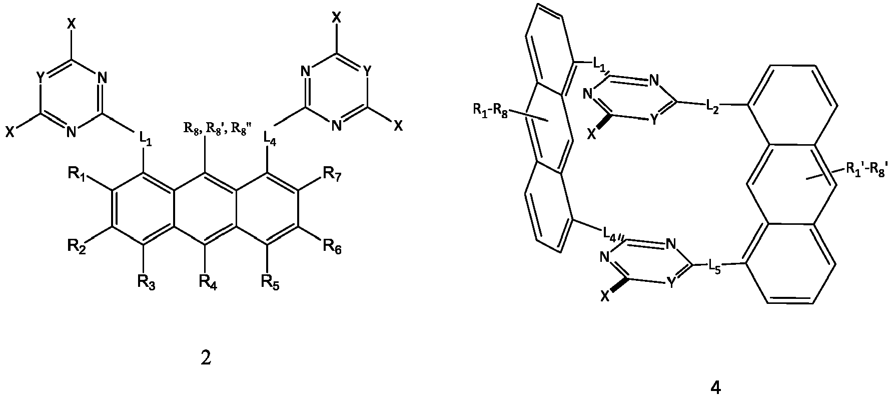 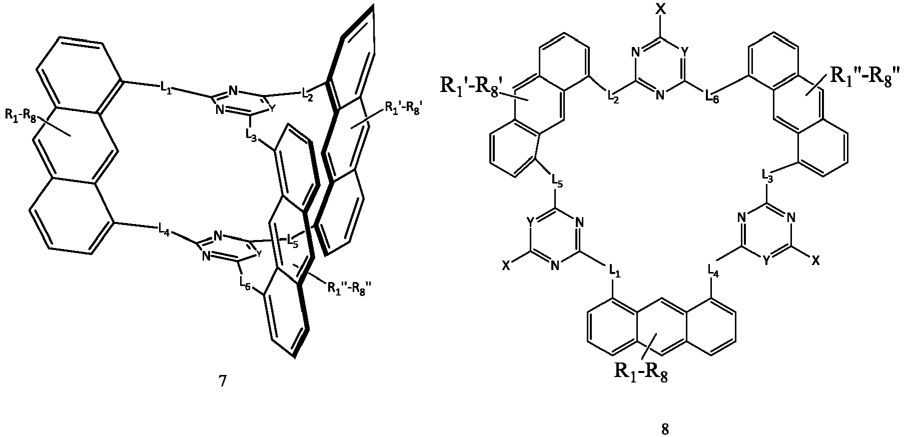 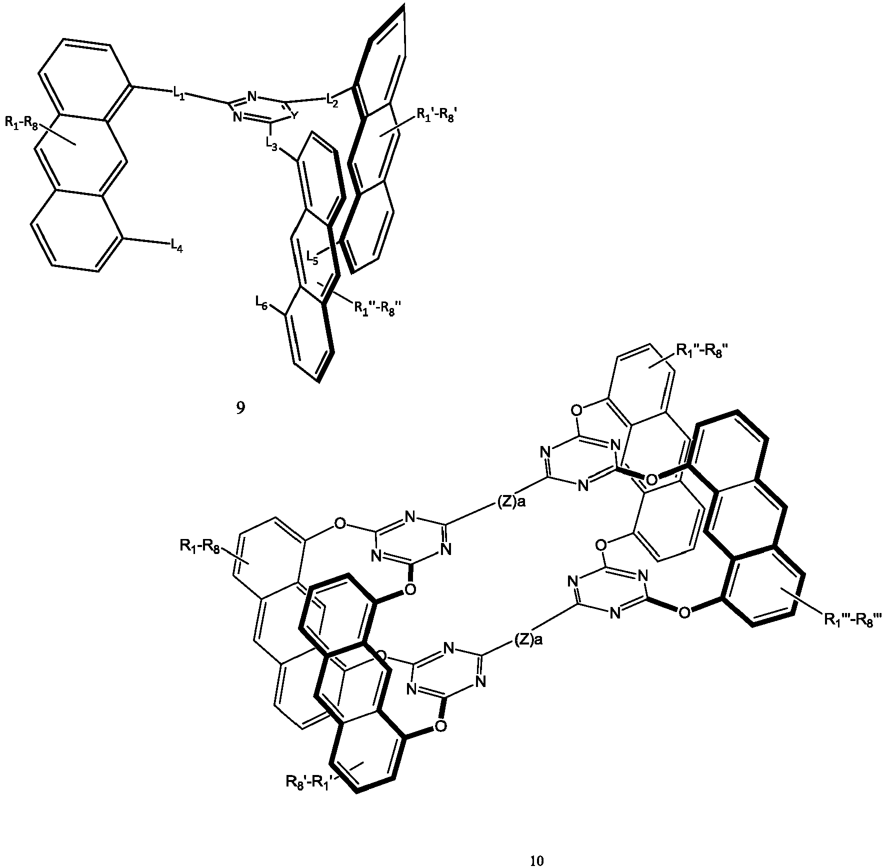 wobei die Reste R₁ bis R₈ der ersten Anthracengruppe, R₁' bis R₈' der zweiten Anthracengruppe, R₁" bis R₈" der dritten Anthracengruppe und R₁''' bis R₈''' der vierten Anthracengruppe in den Formeln 4, 7, 8, 9 und 10 wie folgt angeordnet sind 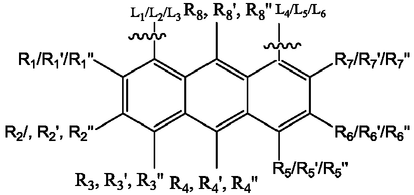 und in den Formeln 2, 4, 7, 8, 9 und 10 R₁, R₁', R₁", R₁''', R₂, R₂', R₂", R₂''', R₃, R₃', R₃", R₃''', R₄, R₄', R₄", R₄''', R₅, R₅', R₅", R₅''', R₆, R₆', R₆", R₆'''^{,} R₇, R₇',R₇", R₇''', R₈, R₈', R₈" und R₈''' unabhängig voneinander Wasserstoff, Deuterium oder eine lineare oder verzweigte Alkylgruppe mit 1 bis 8 Kohlenstoffatomen sind, vorzugsweise ausgewählt aus der Gruppe von Methyl, Ethyl oder Propyl,
jeder von R₁ und R₂, R₁' und R₂', R₁" und R₂", R₁''' und R₂''', R₂ und R₃, R₂' und R₃', R₂" und R₃", R₂''' und R₃''', R₅ und R₆, R₅' und R₆', R₅" und R₆", R₅''' und R₆''', R₆ und R₇, R₆' und R₇', R₆'' und R₇", R₆''' und R₇''' können unabhängig voneinander einen sechsgliedrigen aromatischen Ring bilden, der mit einer linearen oder verzweigten Alkylgruppe mit 1 bis 16 Kohlenstoffatomen substituiert sein kann, vorzugsweise ausgewählt von der Gruppe aus Methyl, Ethyl oder Propylresten, und
L₁, L₂, L₃, L₄, L₅ und L₆ unabhängig voneinander Sauerstoff, Schwefel, Acetylen oder NR₉ sind, und R₉ Wasserstoff oder Methyl ist, und
X entweder Chlor oder Fluor ist,
Y Stickstoff oder CH, vorzugsweise Stickstoff, ist und
Z NH, O, S, Alkin, Alkylen, Arylen, Heteroarylen, Oligo (Ethylenglycol) ist, und
(a) 0 oder 1 ist.

2. Polymer enthaltend als Monomereinheit eine Verbindung der Formel 7, vorzugsweise eine Verbindung der Formel 7a 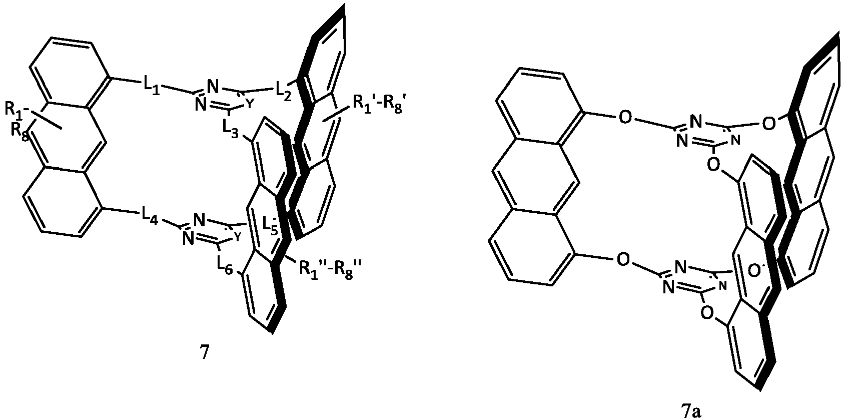 und alle Reste haben die gleiche Definition wie in Anspruch 1.

3. Polymer nach einem der vorangehenden Ansprüche, wobei das Polymer ein Homopolymer ist.

4. Polymer nach einem der Ansprüche 1 bis 2, wobei das Polymer ein Copolymer ist, das eine erste Monomereinheit und eine zweite Monomereinheit enthält, wobei
die erste Monomereinheit ausgewählt ist aus einer Verbindung ausgewählt aus der Gruppe der Formeln 2, 4, 7, 8, 9 und 10, vorzugsweise Verbindung 7a und
die zweite Monomereinheit entweder
(i) ausgewählt ist aus einer Verbindung der Formeln 2, 4, 7, 8, 9 und 10, aber verschieden von der ersten Monomereinheit ist; oder
(ii) eine Verbindung ist, welche die gleiche Formel aufweist wie die erste Monomereinheit, aber wenigstens einer der Reste R₁, R₁', R₁", R₁''', R₂, R₂', R₂", R₂''', R₃, R₃', R₃", R₃''', R₄, R₄', R₄", R₄''', R₅, R₅', R₅", R₅''', R₆, R₆', R₆", R₆''', R₇, R₇', R₇", R₇''', R₈, R₈', R₈", R₈''', L₁, L₂, L₃, L₄, L₅, L₆, X oder Y verschieden ist; oder
(iii) eine monofunktionale oder eine bifunktionale nucleophile Verbindung; oder
(iv) ein Dienophil, vorzugsweise ein Fulleren, am bevorzugtesten ein C60 Fulleren oder ein C70 Fulleren.

5. Polymer nach einem der Ansprüche 1 bis 4, **dadurch gekennzeichnet, dass** das Polymer in einem kristallinien Zustand ist oder eine hybride Kristallstruktur enthält, die Monomereinheit-Domänen und Polymereinheit-Domänen aufweist.

6. Mischung enthaltend das Polymer nach einem der vorangehenden Ansprüche und ein thermoplastisches Polymer ausgewählt aus der Gruppe bestehend aus Polyamiden, Polyestern, Polyproylen, Polyethylen, Polyarylensulfiden, Polyarylenoxiden, Polyimiden, Polyetherketonen, Polyetheretherketonen, Polyoxymethylenen und Polyetherimiden, Polyurethanen, Gummis, Polycyanuraten, Polyaramiden und beliebige Copolymere davon und deren Mischungen.

7. Verfahren zur Herstellung eines Polymers nach einem der Ansprüche 1 bis 5
a) durch Bestrahlung mit ultravioletter Strahlung oder
b) durch thermisch induzierte Polymerisation,
c) durch Druck induzierte Polymerisation wobei die Monomereinheiten in einem uniaxial oder einem biaxial orientierten Zustand sind.

8. Verfahren nach Anspruch 7, wobei vor der Polymerzisation der Monomereinheiten, die Monomereinheiten orientiert werden, indem Kristalle oder Co-Kristalle aus einem Lösungsmittel oder einer Lösungsmittelmischung gebildet werden.

9. Verfahren nach Anspruch 7 oder 8, wobei, vor der Polymerisation der Monomereinheiten, die Monomereinheiten ausgerichtet werden
a. durch Bildung von Kristallen aus einem Lösungsmittel oder einer Lösungsmittelmischung,
b. durch Bildung von Kristallen aus einem Lösungsmittel oder einer Lösungsmittelmischung in einem elektrischen Feld
c. durch Bildung von Co-Kristallen aus einem Lösungsmittel oder einem Lösungsmittelgemisch
d. durch Bildung von Co-Kristallen aus einem Lösungsmittel oder einem Lösungsmittelgemisch in einem elektrischen Feld
e. durch Bildung eines Films an einer Gas-Flüssigkeits-Grenzfläche, oder
f. durch Bildung eines Films aus einem Lösungsmittel oder Lösungsmittelgemisch auf einem festen Substrat, oder
g. durch Sublimieren der Monomereinheiten, um Kristalle zu bilden, oder
h. durch Aufdampfen der Monomereinheiten auf ein Substrat, um eine orientierte Schicht zu bilden, oder
i. durch Sublimieren der Monomereinheiten und Senden dieser in gerichteter Weise auf ein Substrat, wobei eine ausgerichtete Schicht gebildet wird.

10. Verfahren nach einem der Ansprüche 7 bis 9, wobei das Polymer nachträglich durch Funktionalisierung des Polymers verändert wird, vorzugsweise durch Alkylierung von einem oder mehreren Stickstoffatomen in den Triazinkernen oder durch Hydrogenolyse.

11. Verwendung des Polymers nach den Ansprüchen 1 bis 5 oder der Polymermischung nach Anspruch 6 als optoelektronische Vorrichtungen, lichtemittierende Dioden, Laserdioden, organische Transistoren, Kondensatoren, Sensoren, Superkondensatoren, Dielektrika, Dielektrika mit hohem k (High-k-Dielektrika), holographische Vorrichtungen, Energiespeicherungsanwendungen, Batterien, Photovoltaik-Geräte, photonische Kristalleinrichtungen, magnetische Vorrichtungen, ferroelektrische Vorrichtungen, piezoelektrische Vorrichtungen, pyroelektrische Vorrichtungen, transparente leitfähige Beschichtungen, Keimbildner, Klärmittel, nichtlineare optische Vorrichtungen, verschleissfeste Beschichtungen, Schmierbeschichtungen, proteinabweisende Beschichtungen, Katalysatorträger, kratzfeste Beschichtungen, chirale Oberflächen oder als organische Supraleiter, Photokatalyse, Datenspeicheranwendungen, Verpackungen, Membranen, Gastrennmembranen oder Entsalzungsmembranen.

12. Verfahren zur Herstellung des Polymers nach einem der Ansprüche 1 bis 5 durch Rotationsbeschichtung, Tropfgiessen, Tauchbeschichten, Laminieren, Sprühbeschichten, Schmelzextrusion, Reaktionsspritzguss oder Exfoliation.

13. Verfahren zur Herstellung einer Verbindung der Formel 7 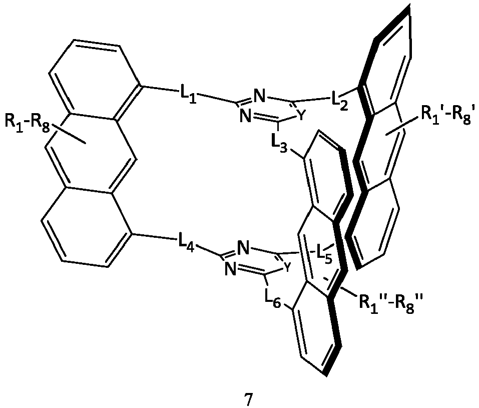 wobei alle Reste die gleiche Definition haben wie in Anspruch 1,
**dadurch gekennzeichnet, dass** es den Schritt umfasst, dass die Verbindung der Formel 3 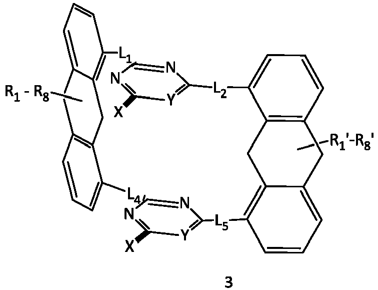 mit der Verbindung B" unter basischen Bedingungen reagiert wird, 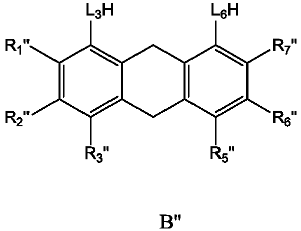 um die Verbindunge der Formel 5 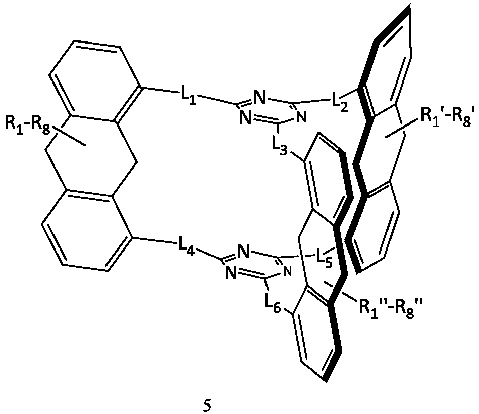 zu bilden.

14. Verfahren nach Anspruch 13, wobei Verbindung 5 in einer Eintopfreaktion gebildet wird, durch Reaktion von 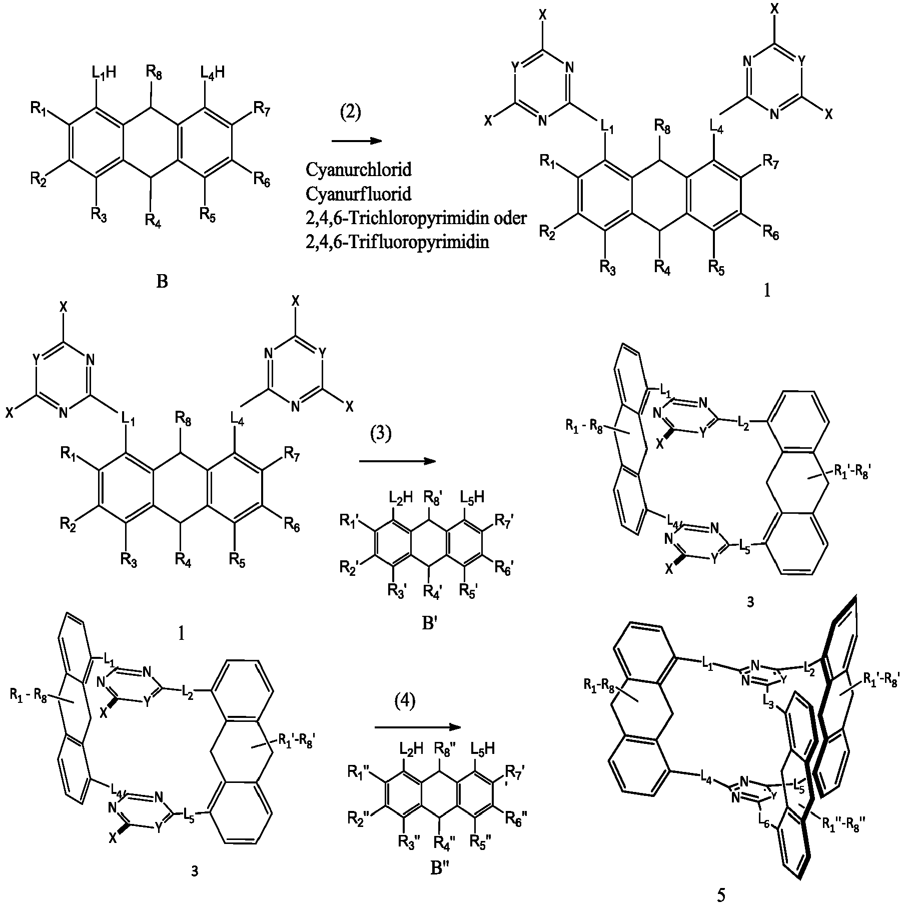 wobei alle Reste die gleiche Definition wie in Anspruch 1 haben,
**dadurch gekennzeichnet, dass** sie in Gegenwart einer Base ausgeführt wird, vorzugweise unter Verwendung von Caesiumcarbonat.

15. Verfahren nach Anspruch 13, wobei die Verbindung der Formel 3 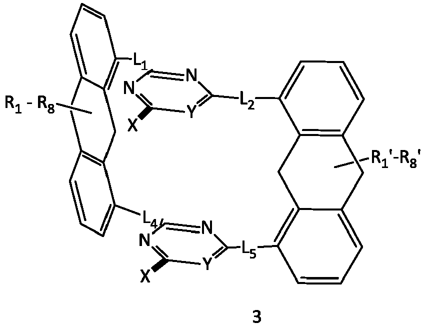 in welche alle Reste die gleiche Definition wie in Anspruch 1 haben,
durch Umsetzung einer Verbindung der Formel 1 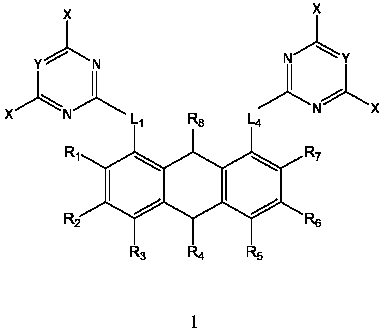 mit der Verbindung B' 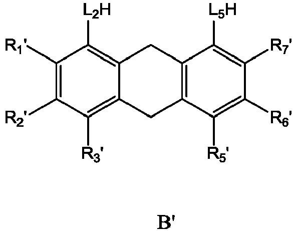 erhalten wird.

16. Monomereinheiten der Formel 2, 4, 7, 8, 9, 10 in einem kristallinen Zustand, wahlweise mit einer Domänenbildung mit einer invertierten Polachse.

17. Verbindung der Formel 7, vorzugsweise Verbindung der Formel 7a in einem kristallinen Zustand.

18. Ein Verfahren zur Induzierung einer Depolymerisation des Polymers nach Anspruch 1 bis 5, vorzugsweise
i. durch thermische Behandlung und/oder
ii. durch UV-Licht und/oder
iii. durch Druck.

19. Verbindung ausgewählt aus der Gruppe aus 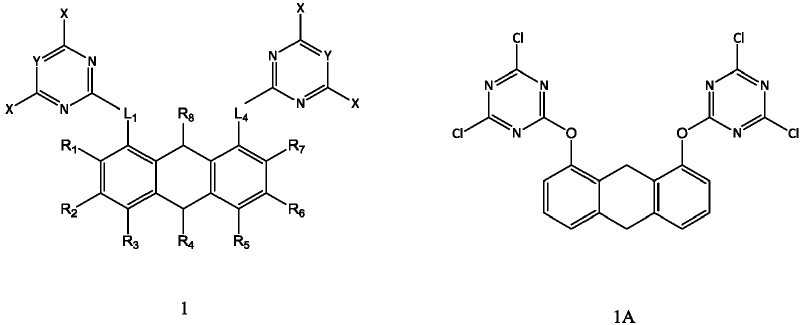 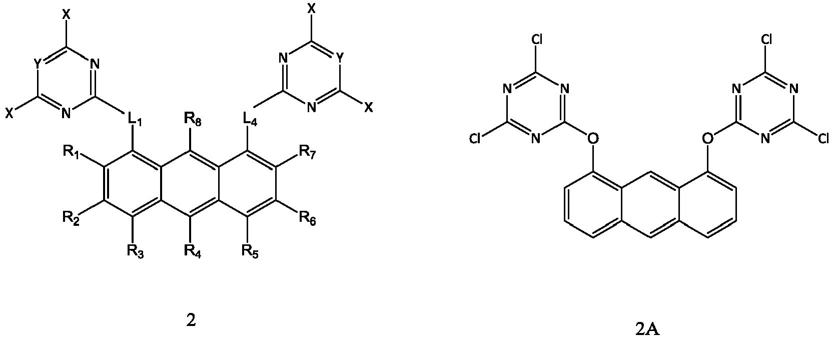 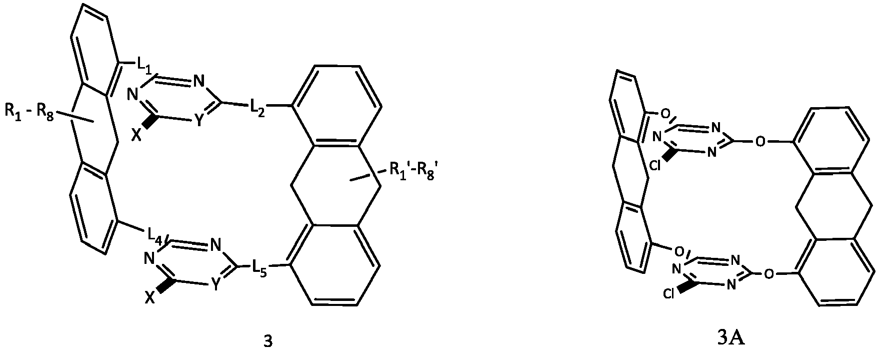 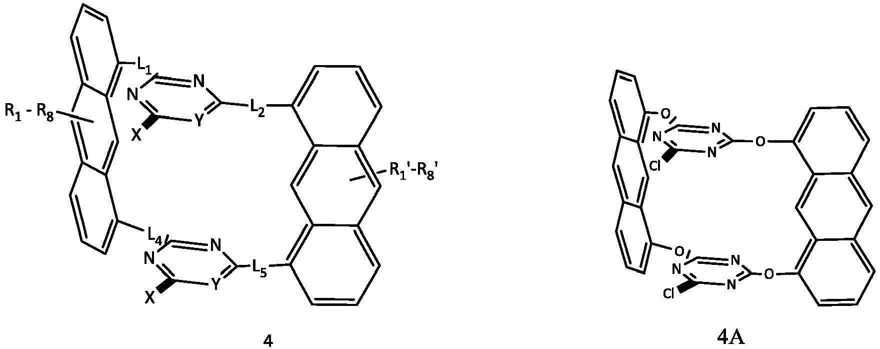 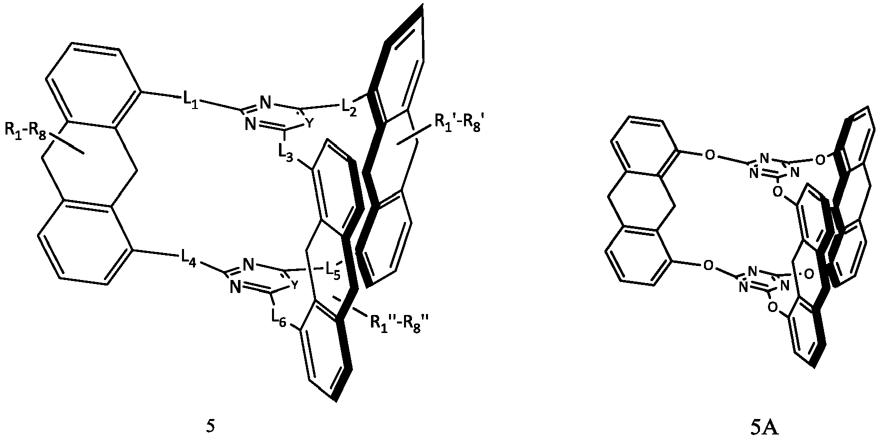 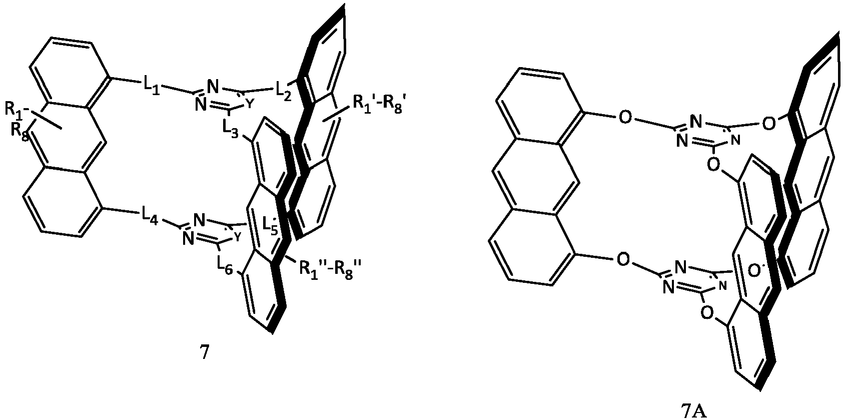 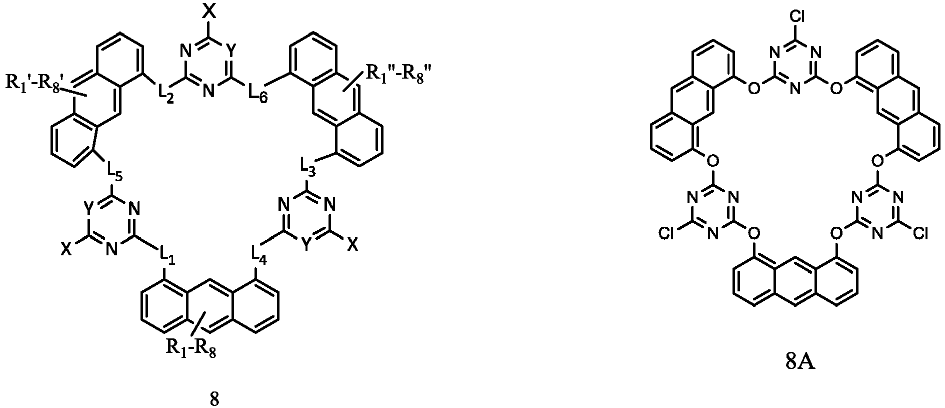 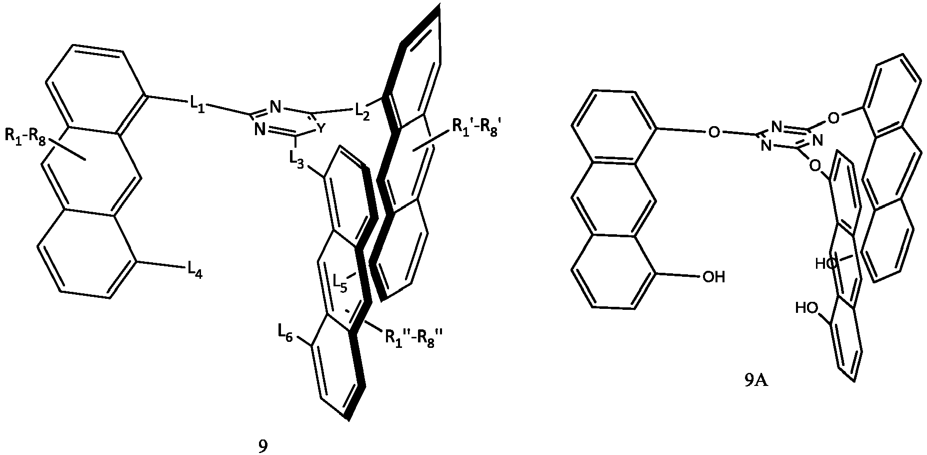 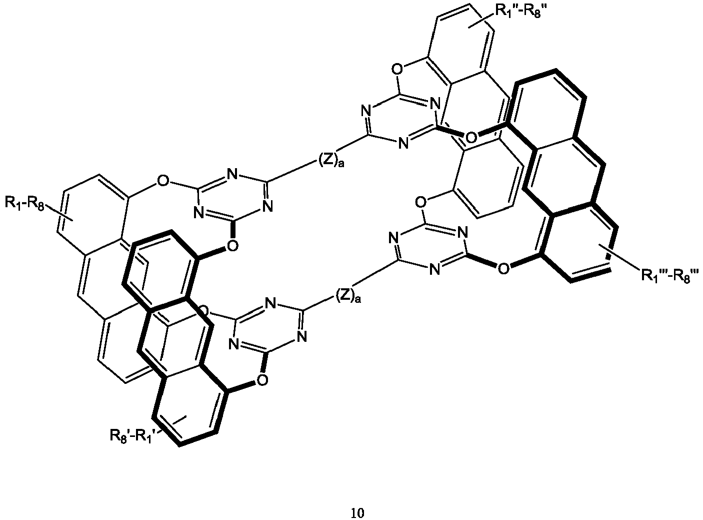 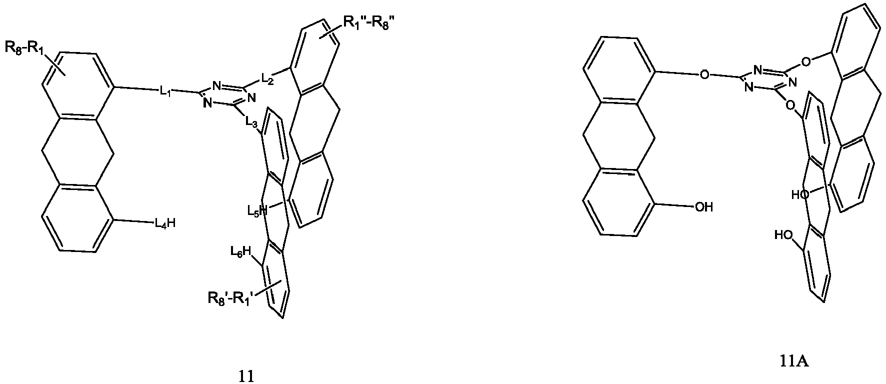 und wobei alle Reste die gleiche Definition wie in Anspruch 1 haben.

## Revendications

1. Polymère comprenant en tant que motif monomère un ou plusieurs composés choisis dans le groupe constitué par 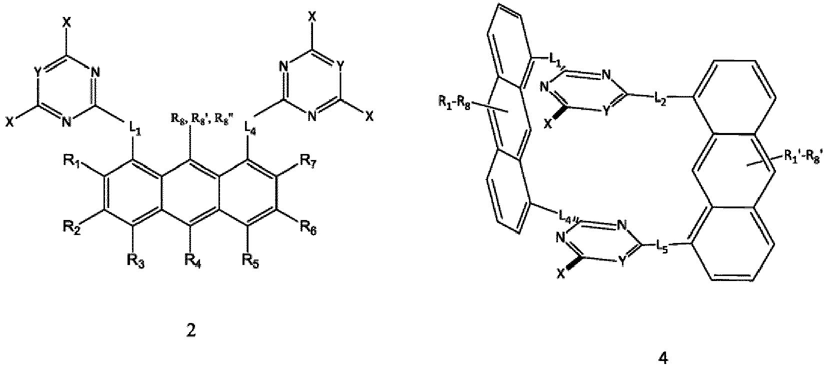 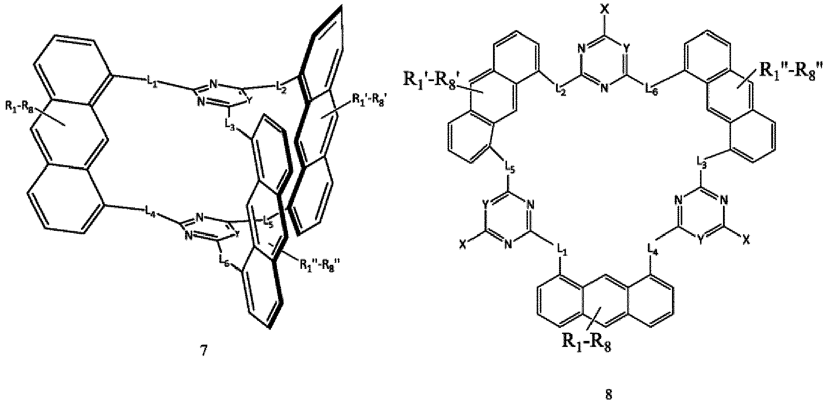 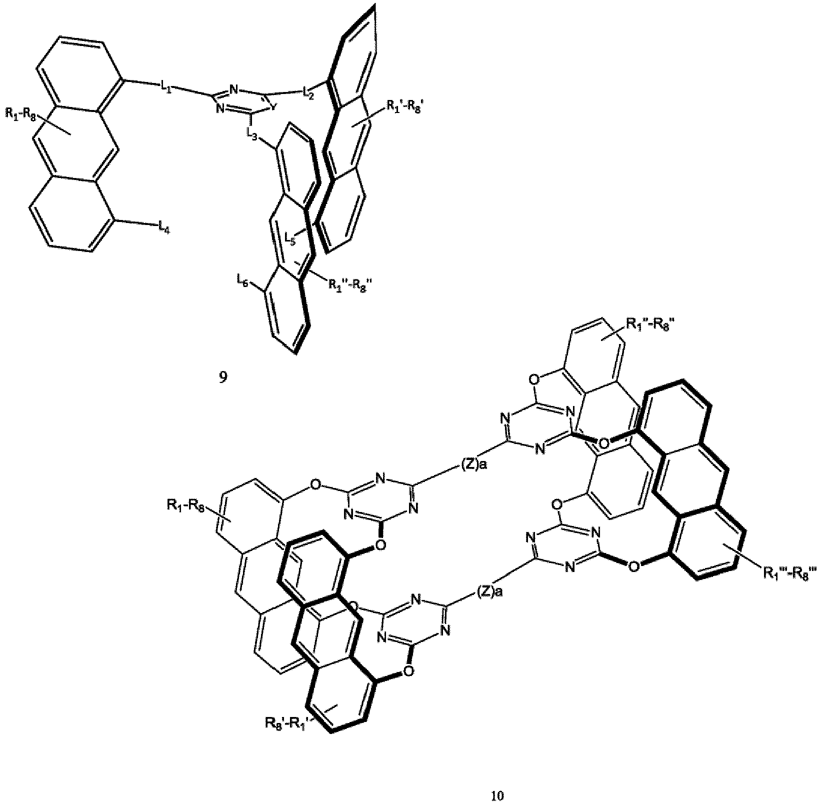 dans lequel les résidus R₁ à R₈ de la première fraction anthracène, R₁' à R₈' de la deuxième fraction anthracène, R₁" à R₈" de la troisième fraction anthracène et R₁''' à R₈''' de la quatrième fraction anthracène, dans les formules 4, 7, 8, 9 et 10 sont placés de la façon suivante 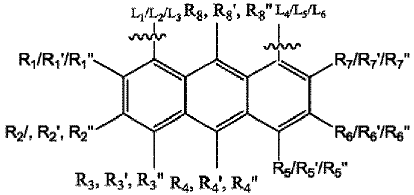 et dans les formules 2, 4, 7, 8, 9 et 10
R₁, R₁', R₁", R₁''', R₂, R₂', R₂", R₂''', R₃, R₃', R₃", R₃''', R₄, R₄', R₄", R₄''', R₅, R₅', R₅", R₅''', R₆, R₆', R₆", R₆''', R₇, R₇', R₇", R₇''', R₈, R₈', R₈" et R₈''' sont chacun indépendamment des autres l'hydrogène, le deutérium ou un groupe alkyle linéaire ou ramifié ayant 1 à 8 atomes de carbone, de préférence choisi dans le groupe des résidus méthyle, éthyle ou propyle,
chacun de R₁ et R₂, R₁' et R₂', R₁" et R₂", R₁"' et R₂''', R₂ et R₃, R₂' et R₃', R₂" et R₃", R₂''' et R₃''', R₅ et R₆, R₅' et R₆', R₅" et R₆", R₅'''et R₆''', R₆ et R₇, R₆' et R₇", R₆" et R₇", R₆''' et R₇''' peuvent former indépendamment les uns des autres un noyau aromatique à six chaînons qui peut éventuellement être substitué par un groupe alkyle linéaire ou ramifié ayant 1 à 16 atomes de carbone, de préférence choisi dans le groupe des résidus méthyle, éthyle ou propyle, et
L₁, L₂, L₃, L₄, L₅ et L₆ sont chacun indépendamment des autres l'oxygène, le soufre, l'acétylène ou NR₉ et R₉ est l'hydrogène ou un résidu méthyle et
X soit le chlore soit le fluor,
Y est l'azote ou CH, de préférence l'azote, et
Z est un résidu NH, O, S, alcyne, alkylène, arylène, hétéroarylène, oligo(éthylèneglycol) et
(a) vaut 0 ou 1.

2. Polymère comprenant en tant que motif monomère un composé de formule 7, de préférence un composé de formule 7a 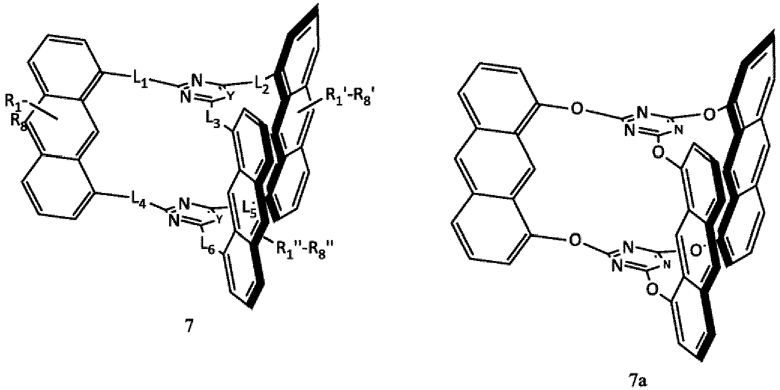 et tous les résidus ont la même définition que dans la revendication 1.

3. Polymère selon l'une quelconque des revendications précédentes, ledit polymère étant un homopolymère.

4. Polymère selon la revendication 1 à 2, ledit polymère étant un copolymère, comprenant un premier motif monomère et un second motif monomère, dans lequel
le premier motif monomère est choisi parmi les composés de formule 2, 4, 7, 8, 9 et 10, de préférence est le composé 7a et
le second motif monomère est soit
(i) choisi parmi les composés de formule 2, 4, 7, 8, 9 et 10 mais est différent du premier motif monomère ; soit
(ii) est un composé ayant la même formule que le premier motif monomère, mais au moins l'un des résidus R₁, R₁', R₁", R₁''', R₂, R₂', R₂", R₂''', R₃, R₃', R₃", R₃''', R₄, R₄', R₄", R₄''', R₅, R₅', R₅", R₅''', R₆, R₆', R₆", R₆''', R₇, R₇', R₇", R₇''', R₈, R₈', R₈", R₈''', L₁, L₂, L₃, L₄, L₅, L₆, X ou Y est différent ; soit
(iii) un composé nucléophile monofonctionnel ou bifonctionnel ; soit
(iv) un diénophile, de préférence un fullerène, de préférence par-dessus tout un fullerène en C60 ou un fullerène en C70.

5. Polymère selon la revendication 1 à 4, **caractérisé en ce que** le polymère est dans un état cristallin ou comprend une structure cristalline hybride, comprenant des domaines de motifs monomères et des domaines de motifs polymères.

6. Mélange comprenant le polymère selon l'une quelconque des revendications précédentes et un polymère thermoplastique choisi dans le groupe constitué par les polyamides, les polyesters, le polypropylène, le polyéthylène, les poly(sulfures d'arylène), les poly(oxydes d'arylène), les polyimides, les polyéthercétones, les polyétheréthercétones, les polyoxyméthylènes et les polyétherimides, les polyuréthanes, les caoutchoucs, les polycyanurates, les polyaramides et de quelconques copolymères de ceux-ci ou mélanges de ceux-ci.

7. Procédé pour la préparation d'un polymère selon les revendications 1 à 5
a) par exposition à un rayonnement ultraviolet ou
b) par polymérisation thermiquement induite,
c) par polymérisation induite par la pression moyennant quoi les motifs monomères sont dans un état orienté de façon uniaxiale ou biaxiale.

8. Procédé selon la revendication 7, dans lequel avant la polymérisation des motifs monomères, les motifs monomères sont orientés par formation de cristaux ou de co-cristaux dans un solvant ou un mélange de solvants.

9. Procédé selon la revendication 7 ou 8, dans lequel, avant la polymérisation des motifs monomères, les motifs monomères sont orientés par
a. par formation de cristaux dans un solvant ou un mélange de solvants
b. par formation de cristaux dans un solvant ou un mélange de solvants dans un champ électrique
c. par formation de co-cristaux dans un solvant ou un mélange de solvants
d. par formation de co-cristaux dans un solvant ou un mélange de solvants dans un champ électrique
e. formation d'un film à une interface gaz-liquide ou
f. formation d'un film dans un solvant ou un mélange de solvants sur un substrat solide ou
g. sublimation des motifs monomères pour former des cristaux ou
h. par dépôt en phase vapeur des motifs monomères sur un substrat pour former une couche orientée ou
i. sublimation des motifs monomères et envoi de ceux-ci de façon dirigée sur un substrat ce qui forme une couche orientée.

10. Procédé selon les revendications 7 à 9, dans lequel le polymère est post-modifié par fonctionnalisation du polymère, de préférence par alkylation d'un ou plusieurs atomes d'azote dans les noyaux triazine ou par hydrogénolyse.

11. Utilisation du polymère selon les revendications 1 à 5 ou du mélange de polymères selon la revendication 6 dans des dispositifs optoélectroniques, des diodes électroluminescentes, des diodes laser, des transistors organiques, des condensateurs, des capteurs, des supercondensateurs, des diélectriques, des diélectriques de constante diélectrique k élevée, des dispositifs holographiques, des applications de stockage d'énergie, des batteries, des dispositifs photovoltaïques, des dispositifs à cristaux photoniques, des dispositifs magnétiques, des dispositifs ferroélectriques, des dispositifs piézoélectriques, des dispositifs pyroélectriques, des revêtements conducteurs transparents, des agents de nucléation, des clarifiants, des dispositifs optiques non linéaires, des revêtements résistants à l'usure, des revêtements lubrifiants, des revêtements répulsifs pour les protéines, des supports de catalyseur, des revêtements de résistance à la rayure, des surfaces chirales ou dans des supraconducteurs organiques, la photocatalyse, des applications de stockage de données, l'emballage, des membranes, des membranes de séparation de gaz ou des membranes de dessalement.

12. Procédé pour la transformation du polymère selon les revendications 1 à 5 par enduction à la tournette, dépôt de goutte, dépôt par trempage, stratification, revêtement par pulvérisation, extrusion à l'état fondu, moulage par injection et réaction ou exfoliation.

13. Procédé pour la préparation d'un composé de formule 7 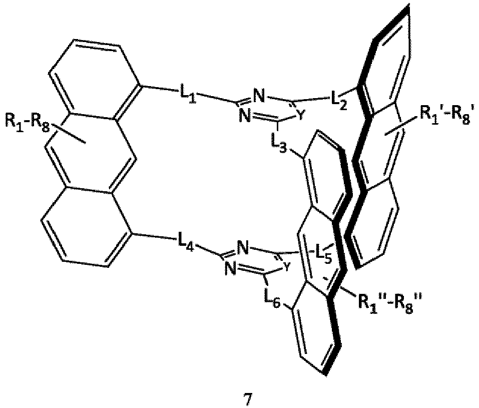 dans lequel tous les résidus ont la même définition que dans la revendication 1,
**caractérisé en ce qu'**il comprend l'étape consistant à faire réagir un composé de formule 3 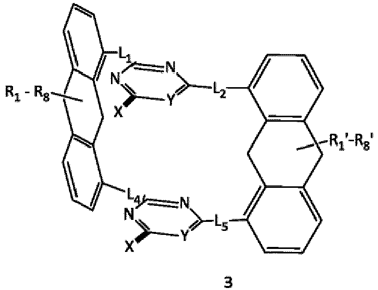 avec un composé B" 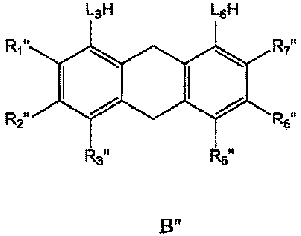 dans des conditions basiques pour former le composé de formule 5

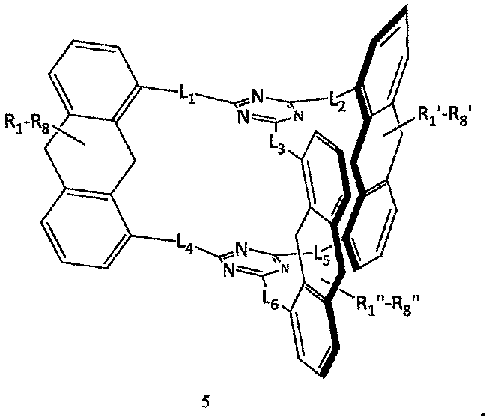

14. Procédé selon la revendication 13, dans lequel le composé 5 est formé dans une réaction monotope par la réaction 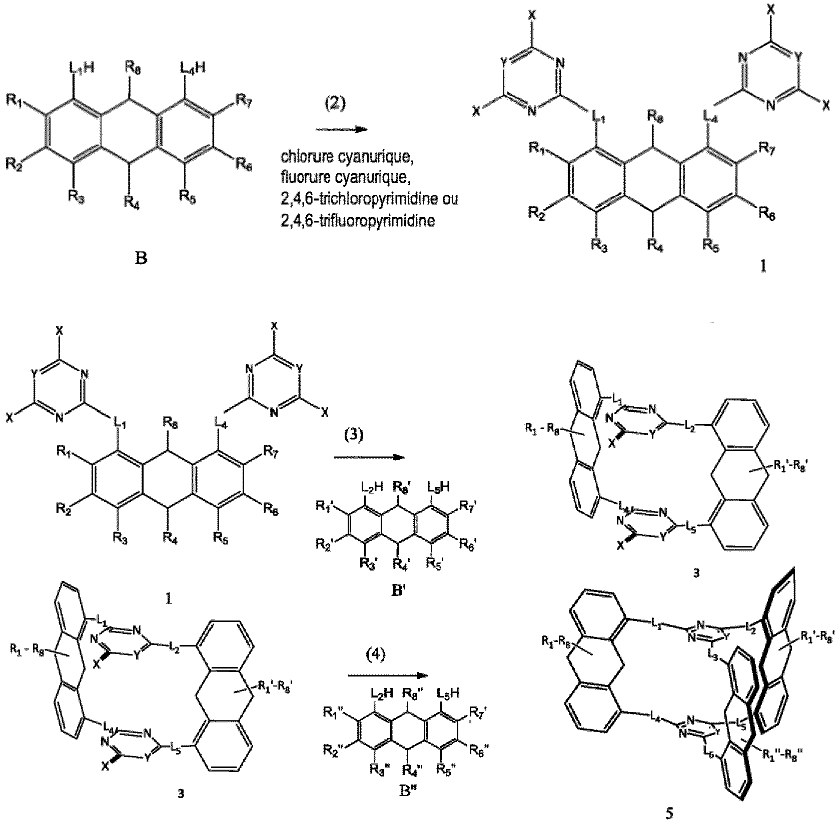 dans lequel tous les résidus ont la même définition que dans la revendication 1,
**caractérisé en ce qu'**il est mis en oeuvre en présence d'une base, de préférence à l'aide de carbonate de césium.

15. Procédé selon la revendication 13, dans lequel un composé de formule 3 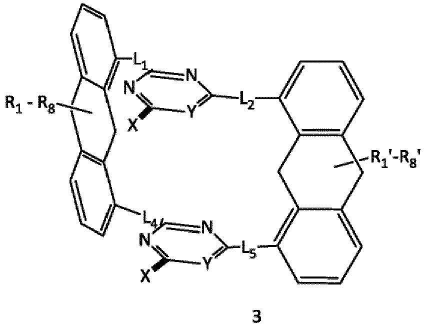 dans lequel tous les résidus ont la même définition que dans la revendication 1,
est obtenu par réaction d'un composé de formule 1 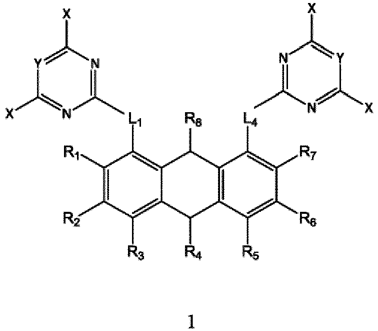 avec un composé B'

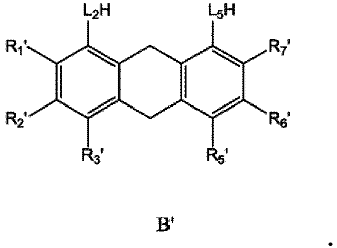

16. Motifs monomères de formule 2, 4, 7, 8, 9, 10 dans un état cristallin, éventuellement ayant une formation de domaines à axes polaires inversés.

17. Composé de formule 7, de préférence composé de formule 7a, dans un état cristallin.

18. Procédé consistant à
induire la dépolymérisation du polymère selon la revendication 1 à 5, de préférence par
i. traitement thermique et/ou
ii. par de la lumière UV et/ou
iii. par de la pression.

19. Composé choisi dans le groupe de 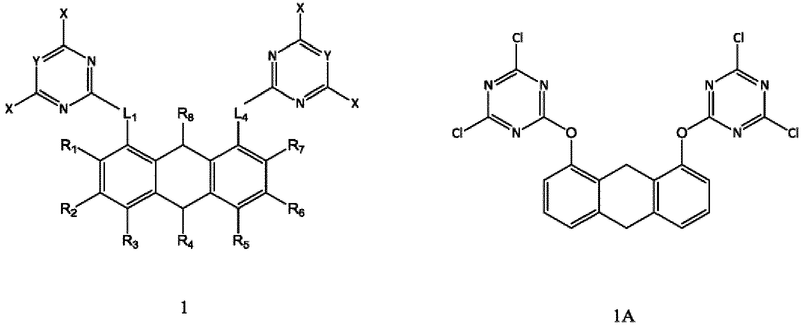 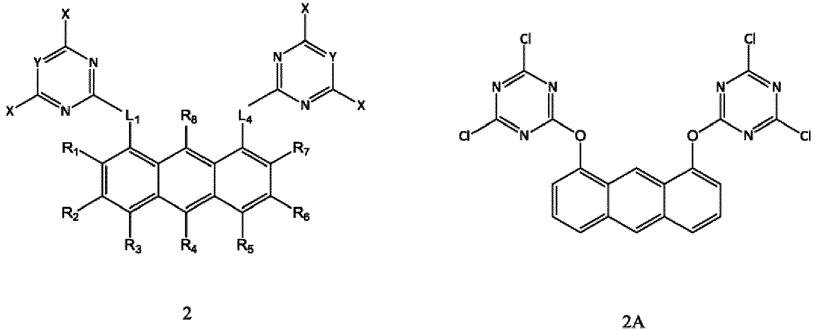 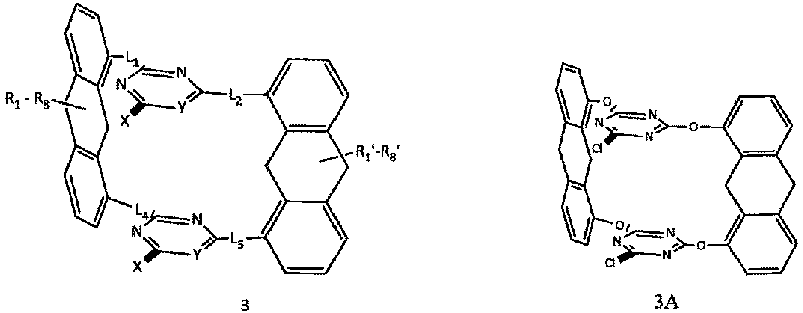 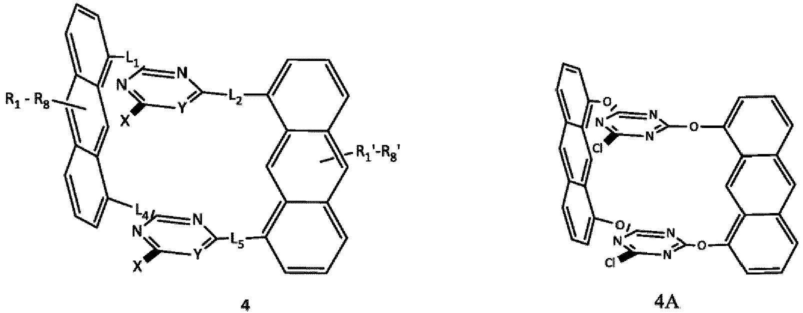 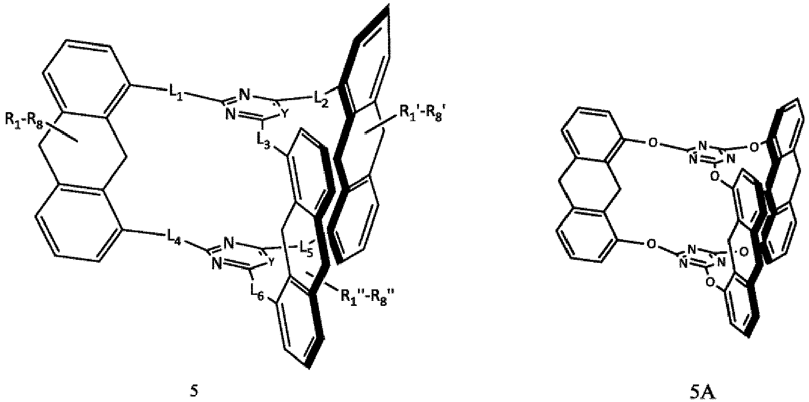 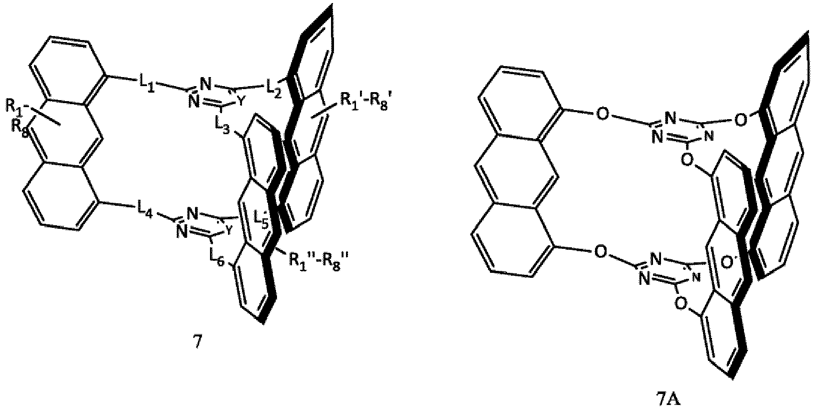 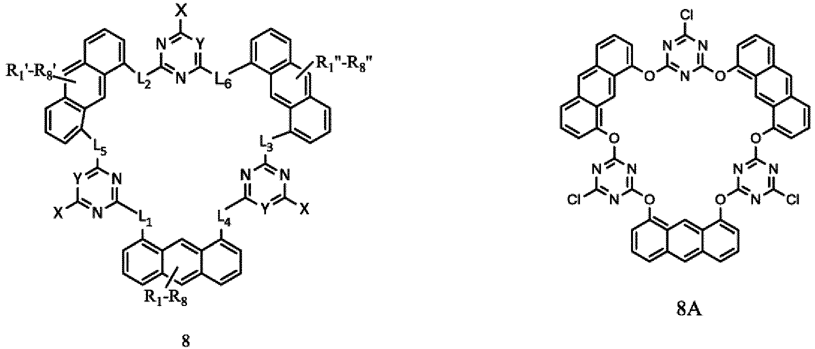 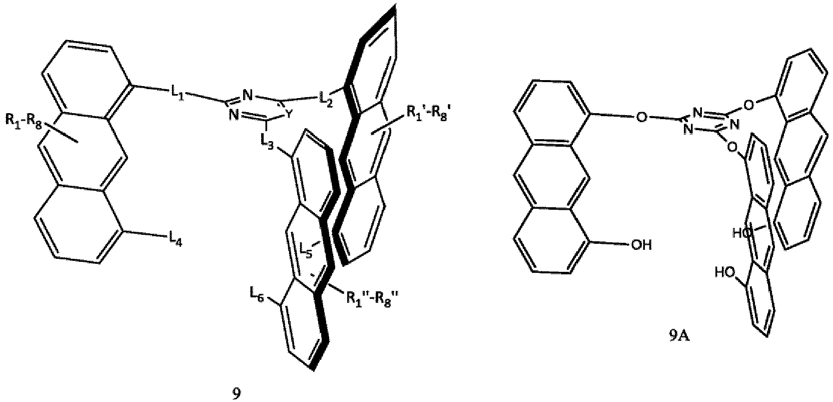 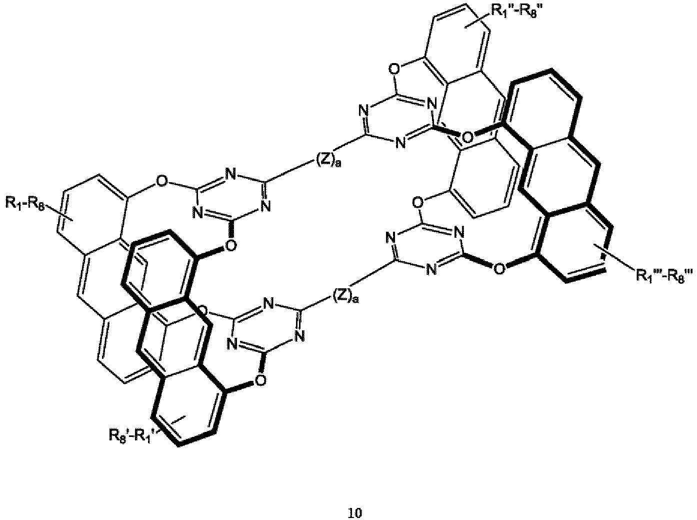 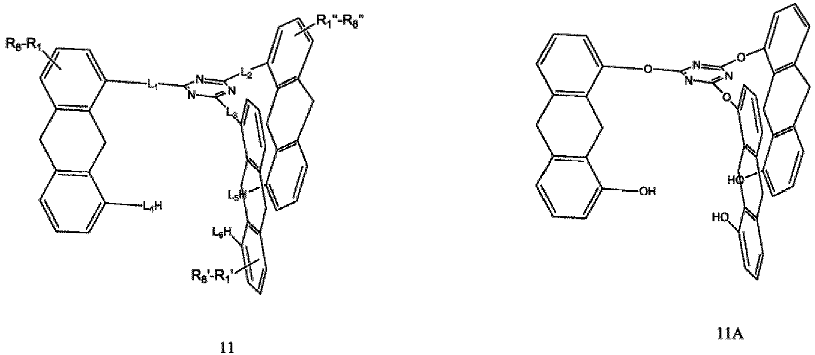 et tous les résidus ont la même définition que dans la revendication 1.
